# EUROPÄISCHE PATENTANMELDUNG

(11) **EP 2 098 120 A2**
(43) Veröffentlichungstag der Anmeldung: **09.09.2009**
(21) Anmeldenummer: 09005139.2
(22) Anmeldetag: 08.12.2005
(51) Int. Cl.: A01N 37/10, A01N 43/08, A01N 43/10, A01N 43/32, A01N 43/40, A01N 43/56, A01N 43/60, A01N 43/78, C07C 233/65, C07D 207/34, C07D 213/82, C07D 231/14, C07D 241/24, C07D 277/56, C07D 307/24, C07D 327/06, C07D 333/38, C07F 7/08

(54) **2-Alkyl-cycloalk(en) yl-carboxamide**

(30) Priorität: 11.12.2004 DE 102004059725
(62) Teilanmeldung aus: 05819324.4
(71) Anmelder: Bayer CropScience AG, 40789 Monheim (DE)
(72) Erfinder: Dunkel, Ralf, 42799 Leichlingen (DE); Elbe, Hans-Ludwig, 42329 Wuppertal (DE); Greul, Jörg, Nico, 42799 Leichlingen (DE); Hartmann, Benoit, 69110 Sainte Foy les Lyon (DE); Gayer, Herbert, 40789 Monheim (DE); Seitz, Thomas, 40764 Langenfeld (DE); Wachendorff-Neumann, Ulrike, 56566 Neuwied (DE); Dahmen, Peter, 41470 Neuss (DE); Kuck, Karl-Heinz, 40764 Langenfeld (DE)

(57) **Zusammenfassung**

Neue 2-Alkyl-cycloalk(en)yl-carboxamide der Formel (I) 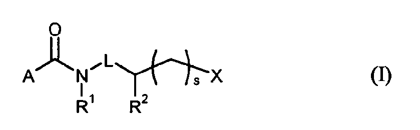 in welcher
X, s, R¹, L, R² und A die in der Beschreibung angegebenen Bedeutungen haben,
mehrere Verfahren zur Herstellung dieser Stoffe und deren Verwendung zur Bekämpfung unerwünschter Mikroorganismen, sowie neue Zwischenprodukte und deren Herstellung.

## Beschreibung

Die vorliegende Erfindung betrifft neue 2-Alkyl-cycloalk(en)yl-carboxamide, mehrere Verfahren zu deren Herstellung und deren Verwendung zur Bekämpfung von unerwünschten Mikroorganismen.

Es ist bereits bekannt, dass zahlreiche Carboxamide fungizide Eigenschaften besitzen (vgl. z.B. WO 98/03495, WO 98/03486 und EP-A 0 589 313). So sind bereits einige 2-Alkyl-cycloalkyl-carboxamide bekannt geworden, wie z.B. *N*-(2-*sec*-Butylcyclohexyl)-2-methyl-4,5-dihydrofuran-3-carbox-amid aus WO 98/03495, *N*-[2-(2-Ethylbutyl)cyclohexyl]-5-fluor-1,3-dimethyl-1H-pyrazol-4-carbox-amid aus WO 98/03486 und *N*-(2-*sec*-Butylcyclohexyl)-2-methyl-4-(trifluormethyl)-1,3-thiazol-5-carboxamid aus EP-A 0 589 313. Die Wirksamkeit dieser Stoffe ist gut, lässt aber in manchen Fällen, z.B. bei niedrigen Aufwandmengen zu wünschen übrig.

Es wurden nun neue 2-Alkyl-cycloalk(en)yl-carboxamide der Formel (I) 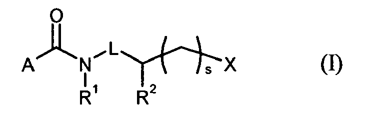 gefunden, in welcher
- X: für -CR³R⁴R⁵ oder-SiR⁴⁹R⁵⁰R⁵¹ steht
- s: für 1 oder 2 steht,
- R¹: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkyl-sulfinyl, C₁-C₄-Halogenalkylsulfonyl, Halogen-C₁-C₄-alkoxy-C₁-C₄-alkyl, C₃-C₈-Halogen-cycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰ steht,
- L: für L¹ oder L² steht,
- L¹: für gegebenenfalls einfach bis vierfach, gleich oder verschieden durch Fluor, Chlor, C₁-C₄-Alkyl oder C₁-C₄-Haloalkyl substituiertes C₃-C₇-Cycloalkyl-1,2-en (C₃-C₇-Cycloallcyl-1,2-diyl) steht,
- L²: für gegebenenfalls einfach bis vierfach, gleich oder verschieden durch Fluor, Chlor, C₁-C₄-Alkyl oder C₁-C₄-Haloalkyl substituiertes Cyclohexenylen (Cyclohexendiyl) steht,
- R²: für Wasserstoff, Halogen, C₁-C₄-Alkyl oder C₁-C₄-Halogenalkyl mit 1 bis 9 Fluor-, Chlor-und/oder Bromatomen steht,
- R³: für Halogen, C₁-C₈-Alkyl oder C₁-C₆-Halogenalkyl mit 1 bis 13 Fluor-, Chlor- und/oder Bromatomen steht,
- R⁴: für Halogen, C₁-C₈-Alkyl oder C₁-C₆-Halogenalkyl mit 1 bis 13 Fluor-, Chlor- und/oder Bromatomen steht,
- R⁵: für Wasserstoff, Halogen, C₁-C₈-Alkyl oder C₁-C₆-Halogenalkyl mit 1 bis 13 Fluor-, Chlor-und/oder Bromatomen steht,
- R³ und R⁴: außerdem gemeinsam mit dem Kohlenstoffatom, an welches sie gebunden sind, einen 3- bis 6-gliedrigen carbocyclischen oder heterocyclischen, gesättigten oder ungesättigten gegebenenfalls durch Halogen, C₁-C₄-Alkyl, C₁-C₄-Alkoxy, C₁-C₄-Haloalkyl oder C₁-C₄-Halo-alkoxy substituierten Ring bilden,
- R⁴⁹ und R⁵⁰: unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
- R⁵¹: für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,
- R⁶: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R⁷ und R⁸: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
- R⁷ und R⁸: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹¹ enthalten kann,
- R⁹ und R¹⁰: unabhängig voneinander für Wasserstoff, C₁-C₈-Alkyl, C₃-C₁₀-Cycloalkyl; C₁-C₈-Halogenalkyl, C₃-C₈-Halogencycloalkyl mit jeweils 1 bis 9 Fluor-, Chlor- und/oder Bromatomen stehen,
- R⁹ und R¹⁰: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹¹ enthalten kann,
- R¹¹: für Wasserstoff oder C₁-C₆-Alkyl steht,
- A: für den Rest der Formel (A1) 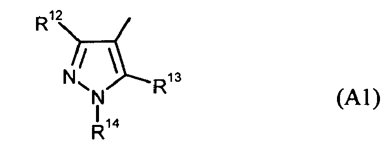 steht, in welcher
- R¹²: für Wasserstoff, Cyano, Halogen, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
- R¹³: für Wasserstoff, Halogen, Cyano, C₁-C₄-Alkyl, C₁-C₄-Alkoxy oder C₁-C₄-Alkylthio steht,
- R¹⁴: für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,
- oder A: für den Rest der Formel (A2) 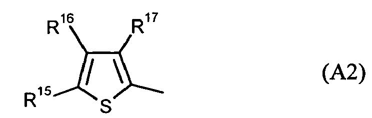 steht, in welcher
- R¹⁵ und R¹⁶: unabhängig voneinander für Wasserstoff, Halogen, C₁-C₄-Alkyl oder C₁-C₄-Halogenalkyl mit 1 bis 5 Halogenatomen steht,
- R¹⁷: für Halogen, Cyano oder C₁-C₄-Alkyl, oder C₁-C₄-Halogenalkyl oder C₁-C₄-Halogenalkoxy mit jeweils 1 bis 5 Halogenatomen steht,
- oder A: für den Rest der Formel (A3) 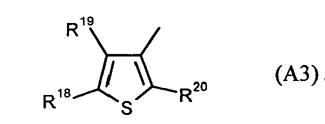 steht, in welcher
- R¹⁸ und R¹⁹: unabhängig voneinander für Wasserstoff, Halogen, C₁-C₄-Alkyl oder C₁-C₄ Halogenalkyl mit 1 bis 5 Halogenatomen steht,
- R²⁰: für Wasserstoff, Halogen, C₁-C₄-Alkyl oder C₁-C₄-Halogenalkyl mit 1 bis 5 Halogenatomen steht,
- oder: A für den Rest der Formel (A4) 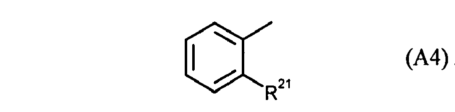 steht, in welcher
- R²¹: für Wasserstoff, Halogen, Hydroxy, Cyano, C₁-C₆-Alkyl, C₁-C₄-Halogenalkyl, C₁-C₄ Halogenalkoxy oder C₁-C₄-Halogenalkylthio mit jeweils 1 bis 5 Halogenatomen steht,
- oder:
- A: für den Rest der Formel (A5) 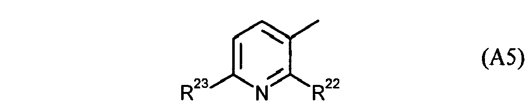 steht, in welcher
- R²²: für Halogen, Hydroxy, Cyano, C₁-C₄-Alkyl, C₁-C₄-Alkoxy, C₁-C₄-Alkylthio, C₁-C₄ Halogenalkyl, C₁-C₄-Halogenalkylthio oder C₁-C₄-Halogenalkoxy mit jeweils 1 bis 5 Halogenatomen steht,
- R²³: für Wasserstoff, Halogen, Cyano, C₁-C₄-Alkyl, C₁-C₄-Alkoxy, C₁-C₄-Alkylthio, C₁ C₄-Halogenalkyl, C₁-C₄-Halogenalkoxy mit jeweils 1 bis 5 Halogenatomen, C₁-C₄ Alkylsulphinyl oder C₁-C₄-Alkylsulphonyl steht,
- oder:
- A: für den Rest der Formel (A6) 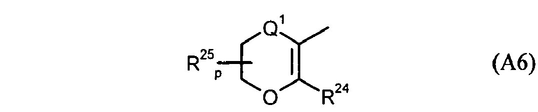 steht, in welcher
- R²⁴: für C₁-C₄-Alkyl oder C₁-C₄-Halogenalkyl mit 1 bis 5 Halogenatomen steht,
- R²⁵: für C₁-C₄-Alkyl steht,
- Q¹: für S (Schwefel), O (Sauerstoff) SO, SO₂ oder CH₂ steht,
- p: für 0, 1 oder 2, wobei R²⁵ für identische oder verschiedene Reste steht, wenn p für 2 steht,
- oder:
- A: für den Rest der Formel (A7) 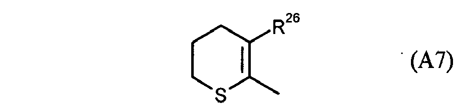 steht, in welcher
- R²⁶: für C₁-C₄-Alkyl oder C₁-C₄-Halogenalkyl mit 1 bis 5 Halogenatomen steht,
- oder:
- A: für den Rest der Formel (A8) 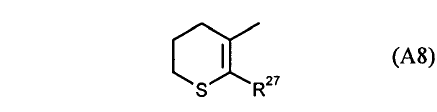 steht, in welcher
- R²⁷: für C₁-C₄-Alkyl oder C₁-C₄-Halogenalkyl mit 1 bis 5 Halogenatomen steht,
- oder:
- A: für den Rest der Formel (A9) 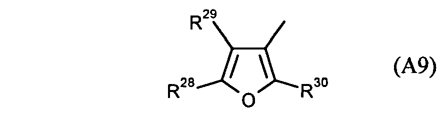 steht, in welcher
- R²⁸ und R²⁹: unabhängig voneinander für Wasserstoff, Halogen, Amino, C₁-C₄-Alkyl oder C₁-C₄-Halogenalkyl mit 1 bis 5 Halogenatomen stehen,
- R³⁰: für Wasserstoff, Halogen, C₁-C₄-Alkyl oder C₁-C₄-Halogenalkyl mit 1 bis 5 Halogenatomen steht,
- oder:
- A: für den Rest der Formel (A10) 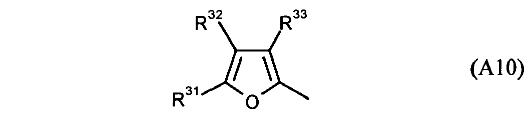 steht, in welcher
- R³¹ und R³²: unabhängig voneinander für Wasserstoff, Halogen, Amino, Nitro, C₁-C₄-Alkyl oder C₁-C₄-Halogenalkyl having 1 bis 5 Halogenatomen stehen,
- R³³: für Wasserstoff, Halogen, C₁-C₄-Alkyl oder C₁-C₄-Halogenalkyl mit 1 bis 5 Halogenatomen steht,
- oder:
- A: für den Rest der Formel (A11) 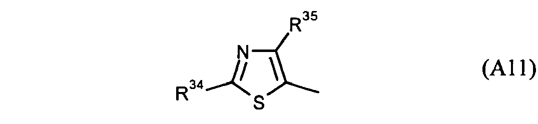 steht, in welcher
- R³⁴: für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
- R³⁵: für Halogen, C₁-C₄-Alkyl oder C₁-C₄-Halogenalkyl mit 1 bis 5 Halogenatomen steht,
- oder:
- A: für den Rest der Formel (A 12) 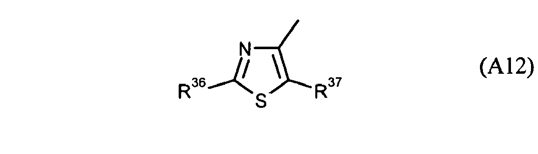 steht, in welcher
- R³⁶: für Wasserstoff, Halogen, Amino, C₁-C₄-Alkylamino, Di-(C₁-C₄-alkyl)ainino, Cyano, C₁-C₄-Alkyl oder C₁-C₄-Halogenalkyl mit 1 bis 5 Halogenatomen steht,
- R³⁷: für Halogen, C₁-C₄-Alkyl oder C₁-C₄-Halogenalkyl mit 1 bis 5 Halogenatomen steht,
- oder:
- A: für den Rest der Formel (A13) 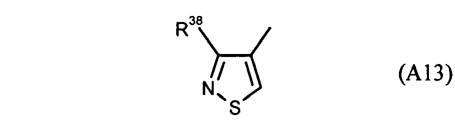 steht, in welcher
- R³⁸: für Halogen, C₁-C₄-Alkyl oder C₁-C₄-Halogenalkyl mit 1 bis 5 Halogenatomen steht,
- oder:
- A: für den Rest der Formel (A14) 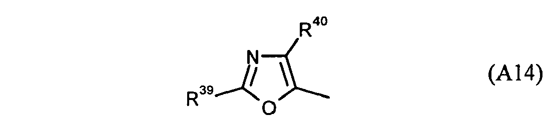 steht, in welcher
- R³⁹: für Wasserstoff oder C₁-C₄-Alkyl steht,
- R⁴⁰: für Halogen oder C₁-C₄-Alkyl steht,
- oder:
- A: für den Rest der Formel (A15) 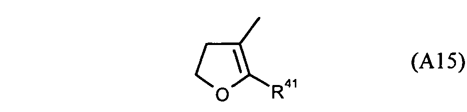
steht, in welcher
- R⁴¹: für C₁-C₄-Alkyl oder C₁-C₄-Halogenalkyl mit 1 bis 5 Halogenatomen steht,
- oder:
- A: für den Rest der Formel (A16) 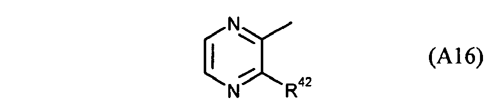 steht, in welcher
- R⁴²: für Wasserstoff, Halogen, C₁-C₄-Alkyl oder C₁-C₄-Halogenalkyl mit 1 bis 5 Halogenatomen steht,
- oder:
- A: für den Rest der Formel (A17) 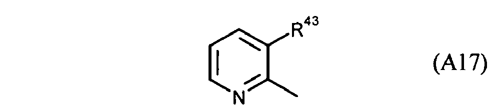 steht, in welcher
- R⁴³: für Halogen, Hydroxy, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
- oder:
- A: für den Rest der Formel (A18) 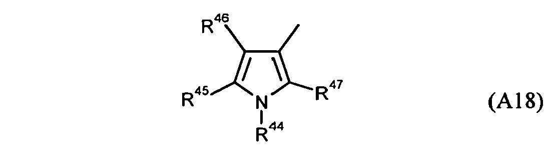 steht, in welcher
- R⁴⁴: für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄-Alkylsulfonyl, Di(C₁-C₄-alkyl)aminosulfonyl, C₁-C₆-Alkylcarbonyl oder jeweils gegebenenfalls substituiertes Phenylsulfonyl oder Benzoyl steht,
- R⁴⁵: für Wasserstoff, Halogen, C₁-C₄-Alkyl oder C₁-C₄-Halogenalkyl mit 1 bis 5 Halogenatomen steht,
- R⁴⁶: für Wasserstoff, Halogen, Cyano, C₁-C₄-Alkyl oder C₁-C₄-Halogenalkyl mit 1 bis 5 Halogenatomen steht,
- R⁴⁷: für Wasserstoff, Halogen, C₁-C₄-Alkyl oder C₁-C₄-Halogenalkyl mit 1 bis 5 Halogenatomen steht,
- oder:
- A: für den Rest der Formel (A19) 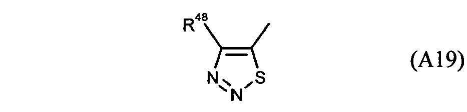 steht, in welcher
- R⁴⁸: für C₁-C₄-Alkyl steht.

Weiterhin wurde gefunden, dass man 2-Alkyl-cycloalk(en)yl-carboxamide der Formel (I) erhält, indem man (a) Carbonsäure-Derivate der Formel (II) 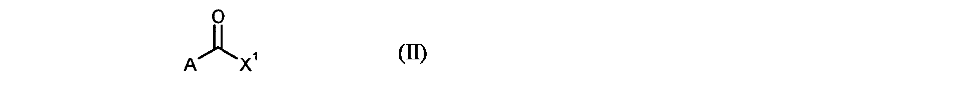 in welcher
A die oben angegebenen Bedeutungen hat und
- X¹: für Halogen oder Hydroxy steht, mit Anilin-Derivaten der Formel (III) 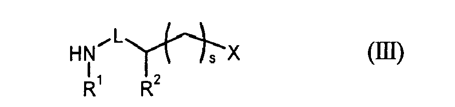 in welcher X, s, R¹, L und R² die oben angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder
(b) 2-Alkyl-cycloalk(en)yl-carboxamide der Formel (I-a) 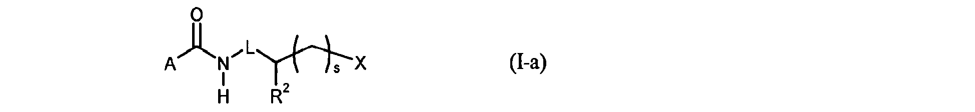 in welcher X, s, L, R² und A die oben angegebenen Bedeutungen haben, mit Halogeniden der Formel (IV)

R^{1A}-X² (IV)

in welcher
- R^{1A}: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈ Halogencycloalkyl mit jeweils 1 bis 9 Fluor-, Chlor- und/oder Bromatomen; Formyl, Forrnyl-C₁-C₃-alkyl, (C₁-C₃-Alkyl)carbonyl-C₁-C₃-alkyl, (C₁-C₃-Alkoxy)carbonyl-C₁ C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰ steht, R⁶, R⁷, R⁸, R⁹ und R¹⁰ die oben angegebenen Bedeutungen haben,
- X²: für Chlor, Brom oder Iod steht, in Gegenwart einer Base und in Gegenwart eines Verdünnungsmittels umsetzt.

Schließlich wurde gefunden, dass die neuen 2-Alkyl-cycloalk(en)yl-carboxamide der Formel (I) sehr gute mikrobizide Eigenschaften besitzen und zur Bekämpfung unerwünschter Mikroorganismen sowohl im Pflanzenschutz als auch im Materialschutz verwendbar sind.

Die erfindungsgemäßen Verbindungen können gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die erfindungsgemäßen 2-Alkyl-cycloalk(en)yl-carboxamide sind durch die Formel (I) allgemein definiert. Bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.
- X: steht bevorzugt für -CR³R⁴R⁵.
- X: steht außerdem bevorzugt für -SiR⁴⁹R⁵⁰R⁵¹.

- s: steht bevorzugt für 1.
- s: steht außerdem bevorzugt für 2.
- s: steht besonders bevorzugt für 1.

- R¹: steht bevorzugt für Wasserstoff, C₁-C₆-Alkyl, C₁-C₄-Alkylsulfmyl, C₁-C₄-Alkylsulfonyl, C₁-C₃-Alkoxy-C₁-C₃-alkyl, C₃-C₆-Cycloalkyl; C₁-C₄-Halogenalkyl, C₁-C₄-Halogenalkylthio, C₁-C₄-Halogenalkylsulfinyl, C₁-C₄-Halogenalkylsulfonyl, Halogen-C₁-C₃-alkoxy-C₁-C₃-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆-Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰.
- R¹: steht besonders bevorzugt für Wasserstoff, Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec- oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂CO₂CH(CH₃)₂,
-(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R⁶, -CONR⁷R⁸ oder -CH₂NR⁹R¹⁰.
- R¹: steht ganz besonders bevorzugt für Wasserstoff, Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(=O)CH₂OCH₃, -C(=O)CO₂CH₃, -C(=O)CO₂CH₂CH₃.
- L: steht bevorzugt für L¹.
- L¹: steht bevorzugt für eine der folgenden Gruppen 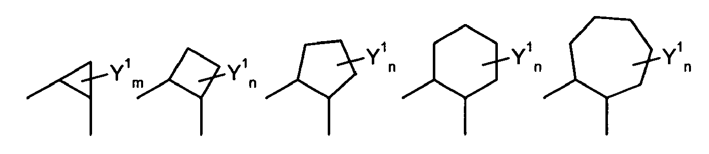 in welchen
- m: für 0, 1 oder 2 steht,
- n: für 0, 1, 2, 3, oder 4 steht,
- Y¹: für Fluor, Chlor, Methyl, Ethyl, n- oder iso-Propyl, n-, iso-, sec- oder tert-Butyl, Trifluormethyl oder Difluormethyl steht, wobei die Reste Y¹ gleich oder verschieden sein können, wenn m bzw. n größer als 1 sind.
- L¹: steht besonders bevorzugt für eine der folgenden Gruppen 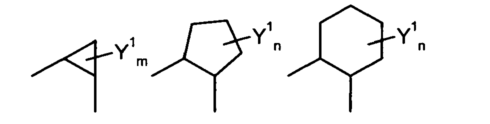 in welchen
- m: für 0, 1 oder 2 steht,
- n: für 0, 1, 2, 3, oder 4 steht,
- Y¹: für Fluor, Chlor, Methyl, Ethyl, n- oder iso-Propyl, n-, iso-, sec- oder tert-Butyl, Trifluormethyl oder Difluormethyl steht, wobei die Reste Y¹ gleich oder verschieden sein können, wenn m bzw. n größer als 1 sind.
- L: steht außerdem bevorzugt für L².
- L²: steht bevorzugt für gegebenenfalls einfach bis vierfach, gleich oder verschieden durch Fluor, Chlor, Methyl, Ethyl, n- oder iso-Propyl, n-, iso-, sec- oder tert-Butyl, Trifluormethyl oder Difluormethyl substituiertes Cyclohexenylen (Cyclohexendiyl), wobei die Doppelbindung in 1,2-Position, 2,3-Position, 3,4-Position, 4,5-Position oder 5,6-Position stehen kann.
- L²: steht besonders bevorzugt für die Gruppe 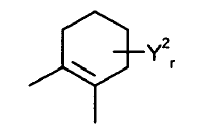 in welcher
- r: für 0 oder 1 steht,
- Y²: für Fluor, Chlor oder Methyl.
- R²: steht bevorzugt für Wasserstoff, Fluor, Chlor, Methyl, Ethyl, n- oder iso-Propyl, oder für jeweils einfach oder mehrfach, gleich oder verschieden durch Fluor, Chlor oder Brom substituiertes Methyl, Ethyl, n- oder iso-Propyl, n-, iso-, sec- oder tert-Butyl.
- R²: steht besonders bevorzugt für Wasserstoff, Fluor, Chlor, Methyl, Ethyl, Trifluormethyl, Difluormethyl, Fluormethyl, Trichlormethyl, Dichlormethyl, Chlormethyl, Chlorfluormethyl, Fluordichlormethyl, Difluorchlormethyl, Pentafluorethyl, 1-Fluorethyl, 2-Fluorethyl, 2,2-Difluorethyl, 2,2,2-Trifluorethyl, 2-Chlor-2-fluorethyl, 2-Chlor-2,2difluorethyl, 2-Chlor-2,2-difluorethyl, 2-Dichlor-2-fluorethyl, 2,2,2-Trichlorethyl, 1-Chlorbutyl, Heptafluor-n-propyl oder Heptafluorisopropyl.
- R²: steht ganz besonders bevorzugt für Wasserstoff, Methyl, Ethyl oder Trifluormethyl.
- R²: steht insbesondere bevorzugt für Wasserstoff oder Methyl.

- R³: steht bevorzugt für Fluor, Chlor, Brom, Methyl, Ethyl, n- oder iso-Propyl, n-, iso-, sec- oder tert-Butyl oder für jeweils einfach oder mehrfach, gleich oder verschieden durch Fluor, Chlor oder Brom substituiertes Methyl, Ethyl, n- oder iso-Propyl, n-, iso-, sec- oder tert-Butyl.
- R³: steht besonders bevorzugt für Fluor, Chlor, Methyl, Ethyl, n- oder iso-Propyl, n-, iso-, secoder tert-Butyl Trifluormethyl, Difluormethyl, Fluormethyl, Trichlormethyl, Dichlormethyl, Chlormethyl, Chlorfluormethyl, Fluordichlormethyl, Difluorchlormethyl, Pentafluorethyl, 1-Fluorethyl, 2-Fluorethyl, 2,2-Difluorethyl, 2,2,2-Trifluorethyl, 2-Chlor-2-fluorethyl, 2-Chlor-2,2difluorethyl, 2-Chlor-2,2-difluorethyl, 2-Dichlor-2-fluorethyl, 2,2,2-Trichlorethyl, 1-Chlorbutyl, Heptafluor-n-propyl oder Heptafluorisopropyl.
- R³: steht ganz besonders bevorzugt für Chlor, Methyl, Ethyl, iso-Propyl oder Trifluormethyl.
- R⁴: steht bevorzugt für Fluor, Chlor, Brom, Methyl, Ethyl, n- oder iso-Propyl, n-, iso-, sec- oder tert-Butyl oder für jeweils einfach oder mehrfach, gleich oder verschieden durch Fluor, Chlor oder Brom substituiertes Methyl, Ethyl, n- oder iso-Propyl, n-, iso-, sec- oder tert-Butyl.
- R⁴: steht besonders bevorzugt für Fluor, Chlor, Methyl, Ethyl, n- oder iso-Propyl, n-, iso-, sec oder tert-Butyl Trifluormethyl, Difluormethyl, Fluormethyl, Trichlormethyl, Dichlormethyl, Chlormethyl, Chlorfluormethyl, Fluordichlormethyl, Difluorchlormethyl, Pentafluorethyl, 1-Fluorethyl, 2-Fluorethyl, 2,2-Difluorethyl, 2,2,2-Trifluorethyl, 2-Chlor-2-fluorethyl, 2-Chlor-2,2difluorethyl, 2-Chlor-2,2-difluorethyl, 2-Dichlor-2-fluorethyl, 2,2,2-Trichlorethyl, 1-Chlorbutyl, Heptafluor-n-propyl oder Heptafluorisopropyl.
- R⁴: steht ganz besonders bevorzugt für Chlor, Methyl, Ethyl, iso-Propyl oder Trifluormethyl.
- R⁵: steht bevorzugt für Wasserstoff, Fluor, Chlor, Brom, Methyl, Ethyl, n- oder iso-Propyl, n-, iso-, sec oder tert-Butyl oder für jeweils einfach oder mehrfach, gleich oder verschieden durch Fluor, Chlor oder Brom substituiertes Methyl, Ethyl, n- oder iso-Propyl, n-, iso-, sec-oder tert-Butyl.
- R⁵: steht besonders bevorzugt für Wasserstoff, Fluor, Chlor, Methyl, Ethyl, n- oder iso-Propyl, n-, iso-, sec- oder tert-Butyl Trifluormethyl, Difluormethyl, Fluormethyl, Trichlormethyl, Dichlormethyl, Chlormethyl, Chlorfluormethyl, Fluordichlormethyl, Difluorchlormethyl, Pentafluorethyl, 1-Fluorethyl, 2-Fluorethyl, 2,2-Difluorethyl, 2,2,2-Trifluorethyl, 2-Chlor-2-fluorethyl, 2-Chlor-2,2difluorethyl, 2-Chlor-2,2-difluorethyl, 2-Dichlor-2-fluorethyl, 2,2,2-Trichlorethyl, 1-Chlorbutyl, Heptafluor-n-propyl oder Heptafluorisopropyl.
- R⁵: steht ganz besonders bevorzugt für Wasserstoff, Chlor, Methyl, Ethyl, iso-Propyl oder Tri-fluormethyl.
- R³ und R⁴: bilden außerdem bevorzugt gemeinsam mit dem Kohlenstoffatom, an welches sie gebunden sind, einen 3- bis 6-gliedrigen carbocyclischen oder heterocyclischen, gesättigten oder ungesättigten gegebenenfalls durch Halogen, Methyl, Ethyl, Methoxy, Trifluormethyl oder Trifluormethoxy substituierten Ring,
- R³ und R⁴: bilden außerdem besonders bevorzugt gemeinsam mit dem Kohlenstoffatom, an welches sie gebunden sind, einen 3-, 5- oder 6-gliedrigen carbocyclischen gesättigten gegebenenfalls durch Methyl, Ethyl oder Trifluormethyl substituierten Ring,
- R³ und R⁴: bilden außerdem ganz besonders bevorzugt gemeinsam mit dem Kohlenstoffatom, an welches sie gebunden sind, einen 6-gliedrigen carbocyclischen ungesättigten gegebenenfalls durch Halogen, Methyl, Ethyl, Methoxy, Trifluormethyl oder Trifluormethoxy substituierten Ring.
- R⁴⁹ und R⁵⁰: stehen unabhängig voneinander bevorzugt für C₁-C₆-Alkyl, C₁-C₆-Alkoxy, C₁-C₃-Alkoxy-C₁-C₃-alkyl oder C₁-C₃-Alkylthio-C₁-C₃-alkyl.
- R⁴⁹ und R⁵⁰: stehen unabhängig voneinander besonders bevorzugt für Methyl, Ethyl, Methoxy, Ethoxy, Methoxymethyl, Ethoxymethyl, Methoxyethyl, Ethoxyethyl, Methylthiomethyl, Ethylthiomethyl, Methylthioethyl oder Ethylthioethyl.
- R⁴⁹ und R⁵⁰: stehen unabhängig voneinander ganz besonders bevorzugt für Methyl, Methoxy, Methoxymethyl oder Methylthiomethyl.
- R⁴⁹ und R⁵⁰: stehen insbesondere bevorzugt jeweils für Methyl.
- R⁵¹: steht bevorzugt für C₁-C₆-Alkyl, C₁-C₆-Alkoxy, C₁-C₃-Alkoxy-C₁-C₃-alkyl, C₁-C₃-Alkylthio-C₁-C₃-alkyl, C₃-C₆-Cycloalkyl, Phenyl oder Benzyl.
- R⁵¹: steht besonders bevorzugt für Methyl, Ethyl, n- oder iso-Propyl, n-, sec-, iso- oder tert-Butyl, Methoxy, Ethoxy, n- oder iso-Propoxy, n-, sec-, iso- oder tert-Butoxy, Methoxymethyl, Ethoxymethyl, Methoxyethyl, Ethoxyethyl, Methylthiomethyl, Ethylthiomethyl, Methylthioethyl, Ethylthioethyl, Cyclopropyl, Phenyl oder Benzyl.
- R⁵¹: steht ganz besonders bevorzugt für Methyl, Ethyl, n- oder iso-Propyl, iso- oder tert-Butyl, Methoxy, iso-Propoxy, iso- oder tert-Butoxy, Methoxymethyl, Methylthiomethyl oder Phenyl.
- R⁵¹: steht insbesondere bevorzugt für Methyl, Ethyl, n- oder iso-Propyl, iso- oder tert-Butyl, Methoxy, iso-Propoxy, iso- oder tert-Butoxy.
- R⁵¹: steht hervorgehoben für Methyl.
- R⁶: steht bevorzugt für Wasserstoff, C₁-C₆-Allcyl, C₁-C₄-Alkoxy, C₁-C₃-Alkoxy-C₁-C₃-alkyl, C₃ C₆-Cycloalkyl; C₁-C₄-Halogenalkyl, C₁-C₄-Halogenalkoxy, Halogen-C₁-C₃-alkoxy-C₁-C₃ alkyl, C₃-C₆-Halogencycloalkyl mit jeweils 1 bis 9 Fluor-, Chlor- und/oder Bromatomen.
- R⁶: steht besonders bevorzugt für Wasserstoff, Methyl, Ethyl, n- oder iso-Propyl, tert-Butyl, Methoxy, Ethoxy, n- oder iso-Propoxy, tert-Butoxy, Methoxymethyl, Cyclopropyl; Trifluormethyl, Trifluormethoxy.
- R⁷ und R⁸: stehen unabhängig voneinander bevorzugt für Wasserstoff, C₁-C₆-Alkyl, C₁-C₃-Alkoxy-C₁ C₃-alkyl, C₃-C₆-Cycloalkyl; C₁-C₄-Halogenalkyl, Halogen-C₁-C₃-alkoxy-C₁-C₃-alkyl, C₃-C₆ Halogencycloalkyl mit jeweils 1 bis 9 Fluor-, Chlor- und/oder Bromatomen.
- R⁷ und R⁸: bilden außerdem gemeinsam mit dem Stickstoffatom, an das sie gebunden sind, bevorzugt einen gegebenenfalls einfach bis vierfach, gleich oder verschieden durch Halogen oder C₁ C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR¹¹ enthalten kann.
- R⁷ und R⁸: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁷ und R^{S}: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹¹ substituiert sein kann.
- R⁹ und R¹⁰: stehen unabhängig voneinander bevorzugt für Wasserstoff, C₁-C₆-Alkyl, C₃-C₆-Cycloalkyl; C₁-C₄-Halogenalkyl, C₃-C₆-Halogencycloalkyl mit jeweils 1 bis 9 Fluor-, Chlor-und/oder Bromatomen.
- R⁹ und R¹⁰: bilden außerdem gemeinsam mit dem Stickstoffatom, an das sie gebunden sind, bevorzugt einen gegebenenfalls einfach oder mehrfach, gleich oder verschieden durch Halogen oder C₁ C₄-Alkyl substituierten gesättigten Heterocyclus mit 5 oder 6 Ringatomen, wobei der Heterocyclus 1 oder 2 weitere, nicht benachbarte Heteroatome aus der Reihe Sauerstoff, Schwefel oder NR¹¹ enthalten kann.
- R⁹ und R¹⁰: stehen unabhängig voneinander besonders bevorzugt für Wasserstoff, Methyl, Ethyl, n-oder iso-Propyl, n-, iso-, sec- oder tert-Butyl, Methoxymethyl, Methoxyethyl, Ethoxymethyl, Ethoxyethyl, Cyclopropyl, Cyclopentyl, Cyclohexyl; Trifluormethyl, Trichlormethyl, Trifluorethyl, Trifluormethoxymethyl.
- R⁹ und R¹⁰: bilden außerdem gemeinsam mit dem Stickstoffatom, an das sie gebunden sind, besonders bevorzugt einen gegebenenfalls einfach bis vierfach, gleich oder verschieden durch Fluor, Chlor, Brom oder Methyl substituierten gesättigten Heterocyclus aus der Reihe Morpholin, Thiomorpholin oder Piperazin, wobei das Piperazin am zweiten Stickstoffatom durch R¹¹ substituiert sein kann.
- R¹¹: steht bevorzugt für Wasserstoff oder C₁-C₄-Alkyl.
- R¹¹: steht besonders bevorzugt für Wasserstoff, Methyl, Ethyl, n- oder iso-Propyl, n-, iso-, sec oder tert-Butyl.
- A: steht bevorzugt für einen der Reste A1, A2, A3, A4, A5, A6, A9, A10, A11, A12, A16, A17 oder A18.
- A: steht besonders bevorzugt für einen der Reste A1, A2, A3, A4, A5, A6, A9, A11, A16, A17, A18.
- A: ganz besonders bevorzugt für den Rest A1.
- A: außerdem ganz besonders bevorzugt für den Rest A2.
- A: außerdem ganz besonders bevorzugt für den Rest A3.
- A: außerdem ganz besonders bevorzugt für den Rest A4.
- A: außerdem ganz besonders bevorzugt für den Rest A5.
- A: außerdem ganz besonders bevorzugt für den Rest A6.
- A: außerdem ganz besonders bevorzugt für den Rest A9.
- A: außerdem ganz besonders bevorzugt für den Rest A11.
- A: außerdem ganz besonders bevorzugt für den Rest A16.
- A: außerdem ganz besonders bevorzugt für den Rest A17.
- A: außerdem ganz besonders bevorzugt für den Rest A18.
- R¹²: steht bevorzugt für Wasserstoff, Cyano, Fluor, Chlor, Brom, Iod, Methyl, Ethyl, iso-Propyl, Methoxy, Ethoxy, Methylthio, Ethylthio, Cyclopropyl, C₁-C₂-Halogenalkyl, C₁-C₂-Halogenalkoxy mit jeweils 1 bis 5 Fluor, Chlor und/oder Bromatomen, Trifluormethylthio, Difluormethylthio, Aminocarbonyl, Aminocarbonylmethyl oder Aminocarbonylethyl.
- R¹²: steht besonders bevorzugt für Wasserstoff, Fluor, Chlor, Brom, Iod, Methyl, Ethyl, isoPropyl, Monofluormethyl, Monofluorethyl, Difluormethyl, Trifluormethyl, Difluorchlormethyl, Trichlormethyl, Dichlormethyl, Cyclopropyl, Methoxy, Ethoxy, Trifluormethoxy, Trichlormethoxy, Methylthio, Ethylthio, Trifluormethylthio oder Difluormethylthio.
- R¹²: steht ganz besonders bevorzugt Wasserstoff, Fluor, Chlor, Brom, Iod, Methyl, iso-Propyl, Monofluormethyl, Monofluorethyl, Difluormethyl, Trifluormethyl, Difluorchlormethyl oder Trichlormethyl.
- R¹²: steht insbesondere bevorzugt für Methyl, Difluormethyl, Trifluormethyl oder 1-Fluorethyl.
- R¹³: steht bevorzugt für Wasserstoff, Fluor, Chlor, Brom, Iod, Methyl, Ethyl, Methoxy, Ethoxy, Methylthio oder Ethylthio.
- R¹³: steht besonders bevorzugt für Wasserstoff, Fluor, Chlor, Brom, Iod oder Methyl.
- R¹³: steht ganz besonders bevorzugt für Wasserstoff, Fluor, Chlor oder Methyl.
- R¹⁴: steht bevorzugt für Wasserstoff, Methyl, Ethyl, n-Propyl, iso-Propyl, C₁-C₂-Halogenalkyl mit 1 bis 5 Fluor, Chlor und/oder Bromatomen, Hydroxymethyl, Hydroxyethyl, Cyclopropyl, Cyclopentyl, Cyclohexyl oder Phenyl.
- R¹⁴: steht besonders bevorzugt für Wasserstoff, Methyl, Ethyl, iso-Propyl, Trifluormethyl, Difluormethyl, Hydroxymethyl, Hydroxyethyl oder Phenyl.
- R¹⁴: steht ganz besonders bevorzugt für Wasserstoff, Methyl, Trifluormethyl oder Phenyl.
- R¹⁴: steht insbesondere bevorzugt für Methyl.
- R¹⁵ und R¹⁶: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹⁵ und R¹⁶: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁵ und R¹⁶: stehen unabhängig voneinander ganz besonders bevorzugt für Wasserstoff, Fluor, Chlor, Brom, Methyl, Ethyl, Difluormethyl, Trifluormethyl oder Trichlormethyl.
- R¹⁵ und R¹⁶: stehen insbesondere bevorzugt jeweils für Wasserstoff.
- R¹⁷: steht bevorzugt für Fluor, Chlor, Brom, Cyano, Methyl, Ethyl, C₁-C₂-Halogenalkyl oder C₁ C₂-Halogenalkoxy mit jeweils 1 bis 5 Fluor, Chlor und/oder Bromatomen.
- R¹⁷: steht besonders bevorzugt für Fluor, Chlor, Brom, Cyano, Methyl, Trifluormethyl, Trifluormethoxy, Difluormethoxy, Difluorchlormethoxy oder Trichlormethoxy.
- R¹⁷: steht ganz besonders bevorzugt für Fluor, Chlor, Brom, Iod, Methyl, Trifluormethyl oder Trifluormethoxy.
- R¹⁷: steht insbesondere bevorzugt für Methyl.
- R¹⁸ und R¹⁹: stehen unabhängig voneinander bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R¹⁸ und R¹⁹: stehen unabhängig voneinander besonders bevorzugt für Wasserstoff, Fluor, Chlor, Brom, Methyl, Ethyl, Difluormethyl, Trifluormethyl, Difluorchlormethyl oder Trichlormethyl.
- R¹⁸ und R¹⁹: stehen unabhängig voneinander ganz besonders bevorzugt für Wasserstoff, Fluor, Chlor, Brom, Methyl, Ethyl, Difluormethyl, Trifluormethyl oder Trichlormethyl.
- R¹⁸ und R¹⁹: stehen insbesondere bevorzugt jeweils für Wasserstoff.
- R²⁰: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R²⁰: steht besonders bevorzugt für Wasserstoff, Fluor, Chlor, Brom, Iod, Methyl oder Trifluormethyl.
- R²⁰: steht ganz besonders bevorzugt für Methyl.
- R²¹: steht bevorzugt für Wasserstoff, Fluor, Chlor, Brom, Iod, Hydroxy, Cyano, C₁-C₄-Alkyl, C₁ C₂-Halogenalkyl, C₁-C₂-Halogenalkoxy oder C₁-C₂-Halogenalkylthio mit jeweils 1 bis 5 Fluor, Chlor und/oder Bromatomen.
- R²¹: steht besonders bevorzugt für Wasserstoff, Fluor, Chlor, Brom, Iod, Hydroxy, Cyano, Methyl, Ethyl, n-Propyl, iso-Propyl, n-Butyl, iso-Butyl, sec-Butyl, tert-Butyl, Difluormethyl, Trifluormethyl, Difluorchlormethyl, Trichlormethyl, Trifluormethoxy, Difluormethoxy, Difluorchlormethoxy, Trichlormethoxy, Trifluormethylthio, Difluormethylthio, Difluorchlormethylthio oder Trichlormethylthio.
- R²¹: steht ganz besonders bevorzugt für Wasserstoff, Fluor, Chlor, Brom, Iod, Methyl, Difluormethyl, Trifluormethyl oder Trichlormethyl.
- R²¹: steht insbesondere bevorzugt für Iod, Methyl, Difluormethyl oder Trifluormethyl.
- R²²: steht bevorzugt für Fluor, Chlor, Brom, Iod, Hydroxy, Cyano, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R²²: steht besonders bevorzugt für Fluor, Chlor, Brom, Iod, Hydroxy, Cyano, Methyl, Ethyl, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Difluormethylthio, Trifluormethylthio, Trifluormethoxy, Difluormethoxy, Difluorchlormethoxy oder Trichlormethoxy.
- R²²: steht ganz besonders bevorzugt für Fluor, Chlor, Brom, Iod, Methyl, Trifluormethyl, Difluormethyl oder Trichlormethyl.
- R²³: steht bevorzugt für Wasserstoff, Fluor, Chlor, Brom, Iod, Cyano, C₁-C₄-Alkyl, Methoxy, Ethoxy, Methylthio, Ethylthio, C₁-C₂-Halogenalkyl oder C₁-C₂-Halogenalkoxy mit jeweils 1 bis 5 Fluor, Chlor und/oder Bromatomen, C₁-C₂-Alkylsulphinyl oder C₁-C₂-Alkylsulphonyl.
- R²³: steht besonders bevorzugt für Wasserstoff, Fluor, Chlor, Brom, Iod, Cyano, n-Propyl, iso-Propyl, n-Butyl, iso-Butyl, sec-Butyl, tert-Butyl, Trifluormethyl, Difluormethyl, Difluorchlormethyl, Trichlormethyl, Methoxy, Ethoxy, Methylthio, Ethylthio, Trifluormethoxy, Difluormethoxy, Difluorchlormethoxy, Trichlormethoxy, Methylsulphinyl oder Methylsulphonyl.
- R²³: steht ganz besonders bevorzugt für Wasserstoff, Fluor, Chlor, Brom, Iod, n-Propyl, isoPropyl, n-Butyl, iso-Butyl, sec-Butyl, tert-Butyl, Trifluormethyl, Difluormethyl, Trichlormethyl, Methylsulphinyl oder Methylsulphonyl.
- R²³: steht insbesondere bevorzugt für Wasserstoff.
- R²⁴: steht bevorzugt für Methyl, Ethyl oder C₁-C₂-Halogenalkyl mit 1 bis 5 Fluor, Chlor und/oder Bromatomen.
- R²⁴: steht besonders bevorzugt für Methyl, Ethyl, Trifluormethyl, Difluormethyl, Difluorchlormethyl oder Trichlormethyl.
- R²⁵: steht bevorzugt für Methyl oder Ethyl.
- R²⁵: steht besonders bevorzugt für Methyl.
- Q¹: steht bevorzugt für S (Schwefel), SO₂ oder CH₂.
- Q¹: steht besonders bevorzugt für S (Schwefel) oder CH₂.
- Q¹: steht ganz besonders bevorzugt für S (Schwefel).
- p: steht bevorzugt für 0 oder 1.
- p: steht besonders bevorzugt für 0.
- R²⁶: steht bevorzugt für Methyl, Ethyl oder C₁-C₂-Halogenalkyl mit 1 bis 5 Fluor, Chlor und/oder Bromatomen.
- R²⁶: steht besonders bevorzugt für Methyl, Ethyl, Trifluormethyl, Difluormethyl, Difluorchlormethyl oder Trichlormethyl.
- R²⁶: steht ganz besonders bevorzugt für Methyl, Trifluormethyl, Difluormethyl oder Trichlormethyl.
- R²⁷: steht bevorzugt für Methyl, Ethyl oder C₁-C₂-Halogenalkyl mit 1 bis 5 Fluor, Chlor und/oder Bromatomen.
- R²⁷: steht besonders bevorzugt für Methyl, Ethyl, Trifluormethyl, Difluormethyl, Difluorchlormethyl oder Trichlormethyl.
- R²⁷: steht ganz besonders bevorzugt für Methyl, Trifluormethyl, Difluormethyl oder Trichlormethyl.
- R²⁸ und R²⁹: stehen unabhängig voneinander bevorzugt für Wasserstoff, Fluor, Chlor, Brom, Amino, Methyl, Ethyl oder C₁-C₂-Halogenalkyl mit 1 bis 5 Fluor, Chlor und/oder Bromatomen.
- R²⁸ und R²⁹: stehen unabhängig voneinander besonders bevorzugt für Wasserstoff, Fluor, Chlor, Brom, Methyl, Ethyl, Trifluormethyl, Difluormethyl, Difluorchlormethyl oder Trichlormethyl.
- R²⁸ und R²⁹: stehen unabhängig voneinander ganz besonders bevorzugt für Wasserstoff, Fluor, Chlor, Brom, Methyl, Trifluormethyl, Difluormethyl oder Trichlormethyl.
- R²⁸ und R²⁹: stehen insbesondere bevorzugt jeweils für Wasserstoff.
- R³⁰: steht bevorzugt für Wasserstoff, Fluor, Chlor, Brom, Iod, Methyl, Ethyl oder C₁-C₂-Halogenalkyl mit 1 bis 5 Fluor, Chlor und/oder Bromatomen.
- R³⁰: steht besonders bevorzugt für Wasserstoff, Fluor, Chlor, Brom, Iod, Methyl, Ethyl, Trifluormethyl, Difluormethyl, Difluorchlormethyl oder Trichlormethyl.
- R³⁰: steht ganz besonders bevorzugt für Wasserstoff, Fluor, Chlor, Brom, Methyl, Trifluormethyl, Difluormethyl oder Trichlormethyl.
- R³⁰: steht insbesondere bevorzugt für Methyl.
- R³¹ und R³²: stehen unabhängig voneinander bevorzugt für Wasserstoff, Fluor, Chlor, Brom, Amino, Nitro, Methyl, Ethyl oder C₁-C₂-Halogenalkyl mit 1 bis 5 Fluor, Chlor und/oder Bromatomen.
- R³¹ und R³²: stehen unabhängig voneinander besonders bevorzugt für Wasserstoff, Fluor, Chlor, Brom, Nitro, Methyl, Ethyl, Trifluormethyl, Difluormethyl, Difluorchlormethyl oder Trichlormethyl.
- R³¹ und R³²: stehen unabhängig voneinander ganz besonders bevorzugt für Wasserstoff, Fluor, Chlor, Brom, Methyl, Trifluormethyl, Difluormethyl oder Trichlormethyl.
- R³¹ und R³²: stehen insbesondere bevorzugt jeweils für Wasserstoff.
- R³³: steht bevorzugt für Wasserstoff, Fluor, Chlor, Brom, Iod, Methyl, Ethyl oder C₁-C₂-Halogenalkyl mit 1 bis 5 Fluor, Chlor und/oder Bromatomen,
- R³³: steht besonders bevorzugt für Wasserstoff, Fluor, Chlor, Brom, Iod, Methyl, Ethyl, Trifluormethyl, Difluormethyl, Difluorchlormethyl oder Trichlormethyl.
- R³³: steht ganz besonders bevorzugt für Wasserstoff, Fluor, Chlor, Brom, Methyl, Trifluormethyl, Difluormethyl oder Trichlormethyl.
- R³³: steht insbesondere bevorzugt für Methyl.
- R³⁴: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R³⁴: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³⁴: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³⁴: steht insbesondere bevorzugt für Amino, Methylamino, Dimethylamino, Methyl oder Trifluormethyl.
- R³⁵: steht bevorzugt für Fluor, Chlor, Brom, Methyl, Ethyl oder C₁-C₂-Halogenalkyl mit 1 bis 5 Fluor, Chlor und/oder Bromatomen.
- R³⁵: steht besonders bevorzugt für Fluor, Chlor, Brom, Methyl, Ethyl, Trifluormethyl, Difluormethyl, Difluorchlormethyl oder Trichlormethyl.
- R³⁵: steht ganz besonders bevorzugt für Fluor, Chlor, Brom, Methyl, Trifluormethyl, Difluormethyl oder Trichlormethyl.
- R³⁵: steht insbesondere bevorzugt für Methyl, Trifluormethyl oder Difluormethyl.
- R³⁶: steht bevorzugt für Wasserstoff, Fluor, Chlor, Brom, Amino, C₁-C₄-Alkylamino, Di(C₁-C₄-alkyl)amino, Cyano, Methyl, Ethyl oder C₁-C₂-Halogenalkyl mit 1 bis 5 Fluor, Chlor und/oder Bromatomen.
- R³⁶: steht besonders bevorzugt für Wasserstoff, Fluor, Chlor, Brom, Amino, Methylamino, Dimethylamino, Cyano, Methyl, Ethyl, Trifluormethyl, Difluormethyl, Difluorchlormethyl oder Trichlormethyl.
- R³⁶: steht ganz besonders bevorzugt für Wasserstoff, Fluor, Chlor, Brom, Amino, Methylamino, Dimethylamino, Methyl, Trifluormethyl, Difluormethyl oder Trichlormethyl.
- R³⁶: steht insbesondere bevorzugt für Amino, Methylamino, Dimethylamino, Methyl oder Trifluormethyl.
- R³⁷: steht bevorzugt für Fluor, Chlor, Brom, Methyl, Ethyl oder C₁-C₂-Halogenalkyl mit 1 bis 5 Fluor, Chlor und/oder Bromatomen.
- R³⁷: steht besonders bevorzugt für Fluor, Chlor, Brom, Methyl, Ethyl, Trifluormethyl, Difluormethyl, Difluorchlormethyl oder Trichlormethyl.
- R³⁷: steht ganz besonders bevorzugt für Fluor, Chlor, Brom, Methyl, Trifluormethyl, Difluormethyl oder Trichlormethyl.
- R³⁷: steht insbesondere bevorzugt für Methyl, Trifluormethyl oder Difluormethyl.
- R³⁸: steht bevorzugt für Fluor, Chlor, Brom, Methyl, Ethyl oder C₁-C₂-Halogenalkyl mit 1 bis 5 Fluor, Chlor und/oder Bromatomen.
- R³⁸: steht besonders bevorzugt für Fluor, Chlor, Brom, Methyl, Ethyl, Trifluormethyl, Difluormethyl, Difluorchlormethyl oder Trichlormethyl.
- R³⁸: steht ganz besonders bevorzugt für Fluor, Chlor, Brom, Methyl, Trifluormethyl, Difluormethyl oder Trichlormethyl.
- R³⁹: steht bevorzugt für Wasserstoff, Methyl oder Ethyl.
- R³⁹: steht besonders bevorzugt für Methyl.
- R⁴⁰: steht bevorzugt für Fluor, Chlor, Brom, Methyl oder Ethyl,
- R⁴⁰: steht besonders bevorzugt für Fluor, Chlor oder Methyl.
- R⁴¹: steht bevorzugt für Methyl, Ethyl oder C₁-C₂-Halogenalkyl mit 1 bis 5 Fluor, Chlor und/oder Bromatomen.
- R⁴¹: steht besonders bevorzugt für Methyl, Ethyl, Trifluormethyl, Difluormethyl, Difluorchlormethyl oder Trichlormethyl.
- R⁴¹: steht ganz besonders bevorzugt für Methyl, Trifluormethyl, Difluormethyl oder Trichlormethyl.
- R⁴¹: steht insbesondere bevorzugt für Methyl oder Trifluormethyl.
- R⁴²: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R⁴²: steht besonders bevorzugt für Wasserstoff, Fluor, Chlor, Brom, Methyl oder Trifluormethyl.
- R⁴³: steht bevorzugt für Fluor, Chlor, Brom, Iod, Hydroxy, C₁-C₄-Alkyl, Methoxy, Ethoxy, Methylthio, Ethylthio, Difluormethylthio, Trifluormethylthio, C₁-C₂-Halogenalkyl oder C₁-C₂-Halogenalkoxy mit jeweils 1 bis 5 Fluor, Chlor und/oder Bromatomen.
- R⁴³: steht besonders bevorzugt für Fluor, Chlor, Brom, Iod, Methyl, Ethyl, n-Propyl, iso-Propyl, n-Butyl, iso-Butyl, sec-Butyl, tert-Butyl, Trifluormethyl, Difluormethyl, Difluorchlormethyl oder Trichlormethyl.
- R⁴³: steht ganz besonders bevorzugt für Fluor, Chlor, Brom, Iod, Methyl, Trifluormethyl, Difluormethyl oder Trichlormethyl.
- R⁴⁴: steht bevorzugt für Wasserstoff, Methyl, Ethyl, C₁-C₂-Halogenalkyl mit 1 bis 5 Fluor-, Chlor-und/oder Bromatomen, C₁-C₂-Alkoxy-C₁-C₂-alkyl, Hydroxymethyl, Hydroxyethyl, Methylsulfonyl oder Dimethylaminosulfonyl.
- R⁴⁴: steht besonders bevorzugt für Wasserstoff, Methyl, Ethyl, Trifluormethyl, Methoxymethyl, Ethoxymethyl, Hydroxymethyl oder Hydroxyethyl.
- R⁴⁴: steht ganz besonders bevorzugt für Methyl oder Methoxymethyl.
- R⁴⁵: steht bevorzugt für Wasserstoff, Fluor, Chlor, Brom, Methyl, Ethyl oder C₁-C₂-Halogenalkyl mit 1 bis 5 Fluor-, Chlor- und/oder Bromatomen.
- R⁴⁵: steht besonders bevorzugt für Wasserstoff, Fluor, Chlor, Brom, Methyl, Ethyl Trifluormethyl, Difluormethyl oder Trichlormethyl.
- R⁴⁵: steht ganz besonders bevorzugt für Wasserstoff oder Methyl.
- R⁴⁶: steht bevorzugt für Wasserstoff, Fluor, Chlor, Brom, Iod, Cyano, Methyl, Ethyl, iso-Propyl oder C₁-C₂-Halogenalkyl mit 1 bis 5 Fluor-, Chlor- und/oder Bromatomen.
- R⁴⁶: steht besonders bevorzugt für Wasserstoff, Fluor, Chlor, Brom, Iod, Cyano, Methyl, Ethyl, iso-Propyl, Trifluormethyl, Difluormethyl, Difluorchlormethyl oder Trichlormethyl.
- R⁴⁶: steht ganz besonders bevorzugt für Wasserstoff, Methyl, Difluormethyl oder Trifluormethyl.
- R⁴⁷: steht bevorzugt für Wasserstoff, Fluor, Chlor, Brom, Methyl, Ethyl oder C₁-C₂-Halogenalkyl mit 1 bis 5 Fluor, Chlor und/oder Bromatomen.
- R⁴⁷: steht besonders bevorzugt für Wasserstoff, Fluor, Chlor, Brom, Iod, Methyl oder Trifluormethyl.
- R⁴⁷: steht ganz besonders bevorzugt für Wasserstoff.
- R⁴⁸: steht bevorzugt für Methyl, Ethyl, n-Propyl oder iso-Propyl.
- R⁴⁸: steht besonders bevorzugt Methyl oder Ethyl.

Bevorzugt sind solche Verbindungen der Formel (I), in welcher alle Reste jeweils die oben genannten bevorzugten Bedeutungen haben.
Besonders bevorzugt sind solche Verbindungen der Formel (I), in welcher alle Reste jeweils die oben genannten besonders bevorzugten Bedeutungen haben. Ganz besonders bevorzugt sind solche Verbindungen der Formel (I), in welcher alle Reste jeweils die oben genannten ganz besonders bevorzugten Bedeutungen haben.

Bevorzugt und jeweils als Teilmenge der oben genannten Verbindungen der Formel (I) zu verstehen sind folgende Gruppen von neuen Carboxamiden:

Gruppe 1: 2-Alkyl-cycloalk(en)yl-carboxamide der Formel (I-g) 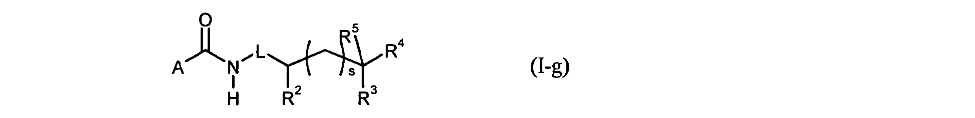 in welcher s, L, R², R³, R⁴, R⁵ und A die oben angegebenen Bedeutungen haben.

Gruppe 2: 2-Alkyl-cycloalk(en)yl-carboxamide der Formel (I-b) 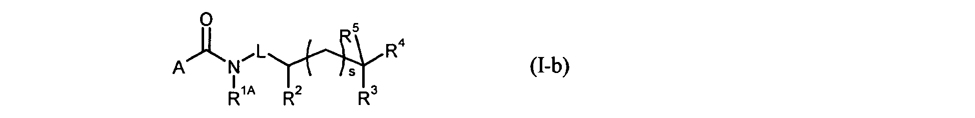 in welcher s, R^{1A}, L, R², R³, R⁴, R⁵ und A die oben angegebenen Bedeutungen haben.
- R^{1A}: steht bevorzugt für C₁-C₆-Alkyl, C₁-C₄-Alkylsulfinyl, C₁-C₄-Alkylsulfonyl, C₁-C₃-Alkoxy-C₁ C₃-alkyl, C₃-C₆-Cycloalkyl; C₁-C₄-Halogenalkyl, C₁-C₄-Halogenalkylthio, C₁-C₄-Halogenal- kylsulfinyl, C₁-C₄-Halogenalkylsulfonyl, Halogen-C₁-C₃-alkoxy-C₁-C₃-alkyl, C₃-C₈-Halogen-cycloalkyl mit jeweils 1 bis 9 Fluor-, Chlor- und/oder Bromatomen; Formyl, Formyl-C₁-C₃ alkyl, (C₁-C₃-Alkyl)carbonyl-C₁-C₃-alkyl, (C₁-C₃-Alkoxy)carbonyl-C₁-C₃-alkyl; Halogen-(C₁ C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₆-Alkyl)carbonyl, (C₁-C₄-Alkoxy)carbonyl, (C₁-C₃-Alkoxy-C₁-C₃-alkyl)carbonyl, (C₃-C₆ Cycloalkyl)carbonyl; (C₁-C₄-Halogenalkyl)carbonyl, (C₁-C₄-Halogenalkoxy)carbonyl, (Halogen-C₁-C₃-alkoxy-C₁-C₃-alkyl)carbonyl, (C₃-C₆-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰.
- R^{1A}: steht besonders bevorzugt für Methyl, Ethyl, n- oder iso-Propyl, n-, iso-, sec- oder tert-Butyl, Pentyl oder Hexyl, Methylsulfinyl, Ethylsulfinyl, n- oder iso-Propylsulfinyl, n-, iso-, sec oder tert-Butylsulfinyl, Methylsulfonyl, Ethylsulfonyl, n- oder iso-Propylsulfonyl, n-, iso-, sec- oder tert-Butylsulfonyl, Methoxymethyl, Methoxyethyl, Ethoxymethyl, Ethoxyethyl, Cyclopropyl, Cyclopentyl, Cyclohexyl, Trifluormethyl, Trichlormethyl, Trifluorethyl, Difluormethylthio, Difluorchlormethylthio, Trifluormethylthio, Trifluormethylsulfinyl, Trifluormethylsulfonyl, Trifluormethoxymethyl; Formyl, -CH₂-CHO, -(CH₂)₂-CHO, -CH₂-CO-CH₃, -CH₂-CO-CH₂CH₃, -CH₂-CO-CH(CH₃)₂, -(CH₂)₂-CO-CH₃, -(CH₂)₂-CO-CH₂CH₃, -(CH₂)₂-CO-CH(CH₃)₂, -CH₂-CO₂CH₃, -CH₂-CO₂CH₂CH₃, -CH₂-CO₂CH(CH₃)₂, -(CH₂)₂-CO₂CH₃, -(CH₂)₂-CO₂CH₂CH₃, -(CH₂)₂-CO₂CH(CH₃)₂, -CH₂-CO-CF₃, -CH₂-CO-CCl₃, -CH₂-CO-CH₂CF₃, -CH₂-CO-CH₂CCl₃, -(CH₂)₂-CO-CH₂CF₃, -(CH₂)₂-CO-CH₂CCl₃, -CH₂-CO₂CH₂CF₃, -CH₂-CO₂CF₂CF₃, -CH₂-CO₂CH₂CCl₃, -CH₂-CO₂CCl₂CCl₃, -(CH₂)₂-CO₂CH₂CF₃, -(CH₂)₂-CO₂CF₂CF₃, -(CH₂)₂-CO₂CH₂CCl₃, -(CH₂)₂-CO₂CCl₂CCl₃; Methylcarbonyl, Ethylcarbonyl, n-Propylcarbonyl, iso-Propylcarbonyl, tert-Butylcarbonyl, Methoxycarbonyl, Ethoxycarbonyl, tert-Butoxycarbonyl, Cyclopropylcarbonyl; Trifluormethylcarbonyl, Trifluormethoxycarbonyl, oder -C(=O)C(=O)R⁶, -CONR⁷R⁸ oder -CH₂NR⁹R¹⁰.
- R^{1A}: steht ganz besonders bevorzugt für Methyl, Methoxymethyl, Formyl, -CH₂-CHO, -(CH₂)₂-CHO, -CH₂-CO-CH₃, -CH₂-CO-CH₂CH₃, -CH₂-CO-CH(CH₃)₂, -C(=O)CHO, -C(=O)C(=O)CH₃, -C(=O)C(O)CH₂OCH₃, -C(=O)CO₂CH₃, -C(=O)CO₂CH₂CH₃.

Gruppe 3: 2-Alkyl-cycloalk(en)yl-carboxamide der Formel (I-c) 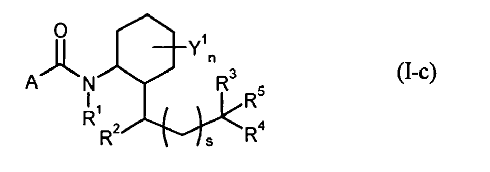 in welcher s, R¹, R², R³, R⁴, R⁵, A, Y¹ und n die oben angegebenen Bedeutungen haben.
Hervorgehoben sind Verbindungen der Formel (I-c), in welcher n für 0 steht. Hervorgehoben sind Verbindungen der Formel (I-c), in welcher R¹ für Wasserstoff steht. Hervorgehoben sind Verbindungen der Formel (I-c), in welcher s für 1 steht.

Gruppe 4: 2-Alkyl-cycloalk(en)yl-carboxamide der Formel (I-d) 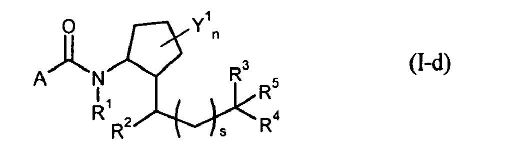 in welcher s, R¹, R², R³, R⁴, R⁵, A, Y¹ und n die oben angegebenen Bedeutungen haben.
Hervorgehoben sind Verbindungen der Formel (I-d), in welcher n für 0 steht.
Hervorgehoben sind Verbindungen der Formel (I-d), in welcher R¹ für Wasserstoff steht. Hervorgehoben sind Verbindungen der Formel (I-d), in welcher s für 1 steht.

Gruppe 5: 2-Alkyl-cycloalk(en)yl-carboxamide der Formel (I-e) 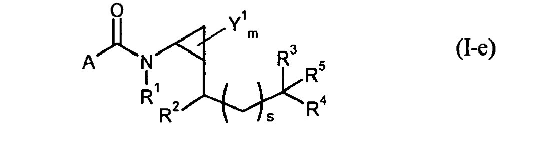 in welcher s, R¹, R², R³, R⁴, R⁵, A, Y¹ und m die oben angegebenen Bedeutungen haben.
Hervorgehoben sind Verbindungen der Formel (I-e), in welcher m für 0 steht.
Hervorgehoben sind Verbindungen der Formel (I-e), in welcher R¹ für Wasserstoff steht. Hervorgehoben sind Verbindungen der Formel (I-e), in welcher s für 1 steht.

Gruppe 6: 2-Alkyl-cycloalk(en)yl-carboxamide der Formel (I-f) 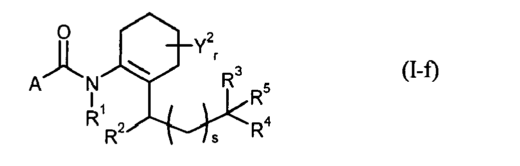 in welcher s, R¹, R², R³, R⁴, R⁵, A, Y² und r die oben angegebenen Bedeutungen haben.
Hervorgehoben sind Verbindungen der Formel (1-f), in welcher r für 0 steht.
Hervorgehoben sind Verbindungen der Formel (I-f), in welcher R¹ für Wasserstoff steht. Hervorgehoben sind Verbindungen der Formel (I-f), in welcher s für 1 steht.

Gruppe 7: 2-Alkyl-cycloalk(en)yl-carboxamide der Formel (I-h) 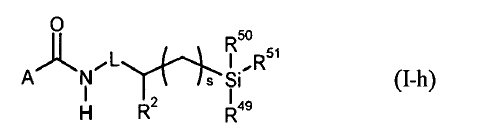 in welcher s, L, R², R⁴⁹, R⁵⁰, R⁵¹ und A die oben angegebenen Bedeutungen haben.

Gruppe 8: 2-Alkyl-cycloalk(en)yl-carboxamide der Formel (I-i) 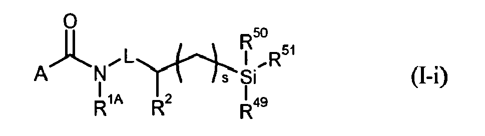 in welcher s, R^{1A}, L, R², R⁴⁹, R⁵⁰, R⁵¹ und A die oben angegebenen Bedeutungen haben.

Gruppe 9: 2-Alkyl-cycloalk(en)yl-carboxamide der Formel (I-k) 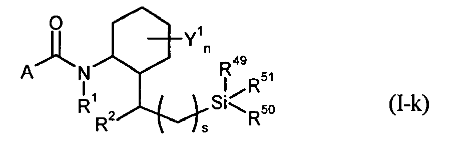 in welcher s, R¹, R², R⁴⁹, R⁵⁰, R⁵¹, A, Y¹ und n die oben angegebenen Bedeutungen haben. Hervorgehoben sind Verbindungen der Formel (I-k), in welcher n für 0 steht.
Hervorgehoben sind Verbindungen der Formel (I-k), in welcher R¹ für Wasserstoff steht. Hervorgehoben sind Verbindungen der Formel (I-k), in welcher s für 1 steht.

Gruppe 10: 2-Alkyl-cycloalk(en)yl-carboxamide der Formel (I-1) 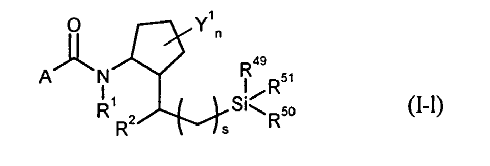 in welcher s, R¹, R², R⁴⁹, R⁵⁰, R⁵¹, A, Y¹ und n die oben angegebenen Bedeutungen haben. Hervorgehoben sind Verbindungen der Formel (I-1), in welcher n für 0 steht.
Hervorgehoben sind Verbindungen der Formel (I-1), in welcher R¹ für Wasserstoff steht.
Hervorgehoben sind Verbindungen der Formel (I-1), in welcher s für 1 steht.

Gruppe 11: 2-Alkyl-cycloalk(en)yl-carboxamide der Formel (I-m) 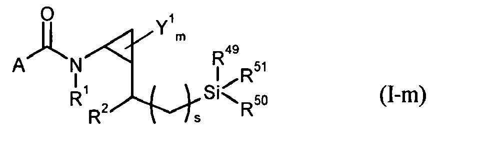 in welcher s, R¹, R², R⁴⁹, R⁵⁰, R⁵¹, A, Y¹ und m die oben angegebenen Bedeutungen haben.
Hervorgehoben sind Verbindungen der Formel (I-m), in welcher m für 0 steht.
Hervorgehoben sind Verbindungen der Formel (I-m), in welcher R¹ für Wasserstoff steht.
Hervorgehoben sind Verbindungen der Formel (I-m), in welcher s für 1 steht.

Gruppe 12: 2-Alkyl-cycloalk(en)yl-carboxamide der Formel (I-n) 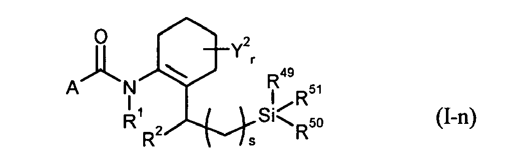 in welcher s, R¹, R², R⁴⁹, R⁵⁰, R⁵¹, A, Y² und r die oben angegebenen Bedeutungen haben.
Hervorgehoben sind Verbindungen der Formel (I-n), in welcher r für 0 steht.
Hervorgehoben sind Verbindungen der Formel (I-n), in welcher R¹ für Wasserstoff steht.
Hervorgehoben sind Verbindungen der Formel (I-n), in welcher s für 1 steht.

Hervorgehoben sind Verbindungen der Formel (I) (und ebenso der Gruppen 2 bis 6 und 8 bis 12), in welcher R¹ bzw. R^{1A} für Formyl steht.
Hervorgehoben sind außerdem Verbindungen der Formel (I) (und ebenso der Gruppen 2 bis 6 und 8 bis 12), in welcher R¹ bzw. R^{1A} für -C(=O)C(=O)R⁶ steht, wobei R⁶ die oben angegebenen Bedeutungen hat.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können. So schließt die Definition Dialkylamino auch eine unsymmetrisch durch Alkyl substituierte Aminogruppe wie z.B. Methyl-ethylamino ein.

Durch Halogen substituierte Reste, wie z.B. Halogenalkyl, sind einfach oder mehrfach halogeniert. Bei mehrfacher Halogenierung können die Halogenatome gleich oder verschieden sein. Halogen steht dabei für Fluor, Chlor, Brom und Iod, insbesondere für Fluor, Chlor und Brom.

Die oben aufgeführten allgemeinen oder in Vorzugsbereichen aufgeführten Restedefinitionen bzw. Erläuterungen können zwischen den jeweiligen Bereichen und Vorzugsbereichen beliebig kombiniert werden. Sie gelten für die Endprodukte sowie für die Vor- und Zwischenprodukte entsprechend.

Insbesondere können die in den Gruppen 1 bis 6 genannten Verbindungen sowohl mit den allgemeinen wie auch mit bevorzugten, besonders bevorzugten usw. Bedeutungen kombiniert werden, wobei auch hier jeweils alle Kombinationen zwischen den Vorzugsbereichen möglich sind.

### Beschreibung der erfindunigsgemäßen Verfahren zum Herstellen der 2-Alkyl-cycloalk(en)yl-carboxamide der Formel (I) sowie der Zwischenprodukte

Verfahren (a) Verwendet man 5-Fluor-1,3-dimethyl-1*H-*pyrazol-4-carbonylchlorid und 2-(1,3-Dimethylbutyl)cyclohexanamin als Ausgangsstoffe, so kann das erfindungsgemäße Verfahren (a) durch das folgende Formelschema veranschaulicht werden:

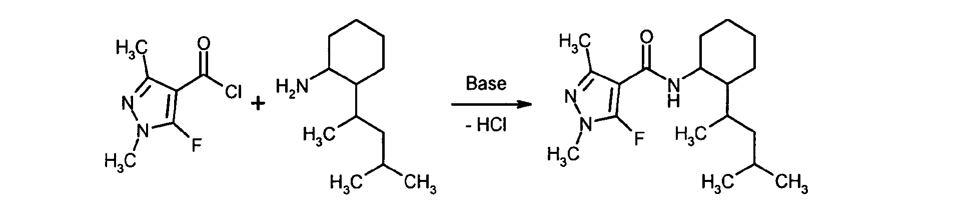

Die zur Durchführung des erfindungsgemäßen Verfahrens (a) als Ausgangsstoffe benötigten Carbonsäure-Derivate sind durch die Formel (II) allgemein definiert. In dieser Formel (II) steht A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diesen Rest angegeben wurden. X¹ steht bevorzugt für Chlor, Brom oder Hydroxy.

Die Carbonsäure-Derivate der Formel (II) sind bekannt und/oder lassen sich nach bekannten Verfahren herstellen (vgl. WO 03/066609, WO 03/066610, EP-A 0 545 099, EP-A 0 589 301, EP-A 0 589 313 und US 3,547,917).

Die zur Durchführung des erfindungsgemäßen Verfahrens (a) als Ausgangsstoffe weiterhin benötigten Anilin-Derivate sind durch die Formel (III) allgemein definiert. In dieser Formel (III) haben X, s, R¹, L und R² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) für diese Reste bzw. diesen Index als bevorzugt, besonders bevorzugt bzw. ganz besonders bevorzugt angegeben wurden.

Die Anilin-Derivate der Formel (III) sind teilweise bekannt oder können nach bekannten Verfahren erhalten werden (vgl. z.B. EP-A 0 589 313).

Es ist auch möglich, zunächst Anilin-Derivate der Formel (III-a) 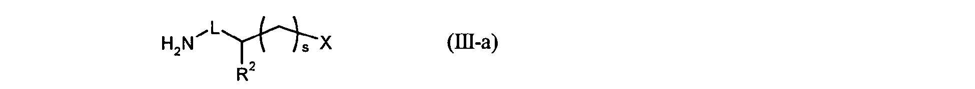 in welcher X, s, L und R² die oben angegebenen Bedeutungen haben, herzustellen und diese gegebenenfalls anschließend mit Halogeniden der Formel (IV)

R^{1A}-X² (IV)

in welcher R^{3A} und X⁴ die oben angegebenen Bedeutungen haben, in Gegenwart einer Base und in Gegenwart eines Verdünnungsmittels umzusetzen. [Die Reaktionsbedingungen des erfindungsgemäßen (b) gelten entsprechend.] Anilin-Derivate der Formel (III) werden auch erhalten, indem man (c) cyclische Ketone der Formel (V) 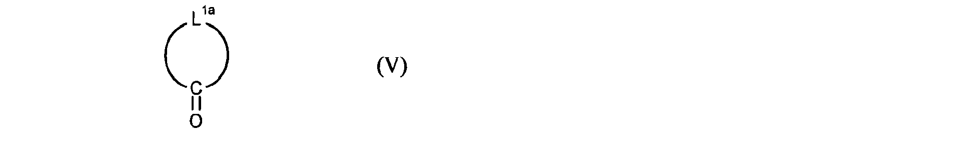 in welcher
- L^{1a}: für gegebenenfalls einfach bis vierfach, gleich oder verschieden durch Fluor, Chlor, C₁C₄-Alkyl oder C₁-C₄-Haloalkyl substituiertes C₂-C₆-Alkylen (C₂-C₆-Alkandiyl) steht, zunächst mit Carbonyl-Verbindungen der Formel (VI) 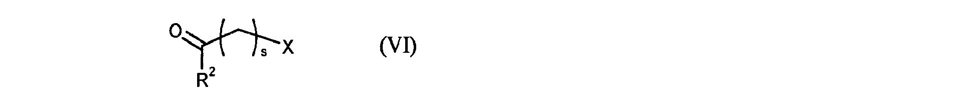 in welcher X, s und R² die oben angegebenen Bedeutungen haben, in Gegenwart einer Base zu den Verbindungen der Formeln (VIIa) und (VIIb) 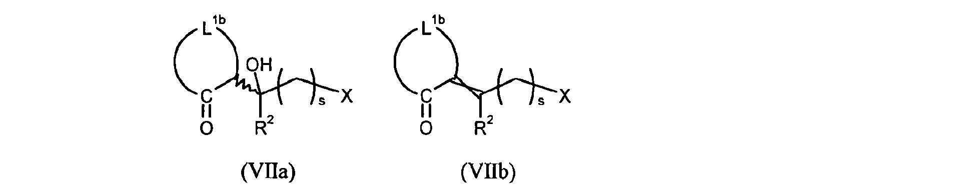 in welcher
- X,: S und R² die oben angegebenen Bedeutungen haben und
- L^{1b}: für gegebenenfalls einfach bis vierfach, gleich oder verschieden durch Fluor, Chlor, C₁ C₄-Alkyl oder C₁-C₄-Haloalkyl substituiertes C₁-C₅-Alkylen (C₁-C₅-Alkandiyl) steht,
umsetzt und diese dann nach üblichen Methoden reduktiv aminiert.

Die zur Durchführung des erfindungsgemäßen Verfahrens (c) als Ausgangsstoffe benötigten cyclischen Ketone sind durch die Formel (V) allgemein definiert. In dieser Formel (V) steht L^{1a} bevorzugt für gegebenenfalls m-fach durch Y¹ substituiertes -(CH₂)₂- oder für jeweils gegebenenfalls n-fach durch Y¹ substituiertes -(CH₂)₃-, -(CH₂)₄-, -(CH₂)₅- oder -(CH₂)₆-, wobei m, n und Y¹ die oben angegebenen Bedeutungen haben. L^{1a} steht besonders bevorzugt für gegebenenfalls m-fach durch Y¹ substituiertes -(CH₂)₂- oder für jeweils gegebenenfalls n-fach durch Y¹ substituiertes -(CH₂)₄- oder -(CH₂)₅-, wobei m, n und Y¹ die oben angegebenen Bedeutungen haben.

Die zur Durchführung des erfindungsgemäßen Verfahrens (c) weiterhin als Ausgangsstoffe benötigten Carbonyl-Verbindungen sind durch die Formel (VI) allgemein definiert. In dieser Formel (VI) haben X, s und R² bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die bei der Durchführung des erfindungsgemäßen Verfahrens (c) als Zwischenprodukte entstehenden Verbindungen sind durch die Formel (VIIa) und (VIIb) allgemein definiert. In diesen Formeln (VIIa) und (VIIb) steht L^{1b} bevorzugt für gegebenenfalls m-fach durch Y¹ substituiertes -(CH₂)- oder für jeweils gegebenenfalls n-fach durch Y¹ substituiertes -(CH₂)₂-, -(CH₂)₃-, -(CH₂)₄- oder -(CH₂)₅-, wobei m, n und Y¹ die oben angegebenen Bedeutungen haben. L^{1b} steht besonders bevorzugt für gegebenenfalls m-fach durch Y¹ substituiertes -(CH₂)- oder für jeweils gegebenenfalls n-fach durch Y¹ substituiertes -(CH₂)₃- oder -(CH₂)₄-, wobei m, n und Y¹ die oben angegebenen Bedeutungen haben. X, s und R² hat bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Cyclischen Ketone der Formel (V) und Carbonyl-Verbindungen der Formel (VI) sind bekannt oder können durch Literatur bekannte Verfahren hergestellt werden (Organic Letters 2001, Vol. 3, 573; Tetrahedron Letters 42 (2001) 4257).

### Verfahren (b)

Verwendet man *N-*[2-(1,3-Dimethylbutyl)cyclohexyl]-5-fluor-1,3-dimethyl-1*H-*pyrazol-4-carboxamid und Acetylchlorid als Ausgangsstoffe, so kann der Verlauf des erfindungsgemäßen Verfahrens (b) durch das folgende Formelschema veranschaulicht werden:

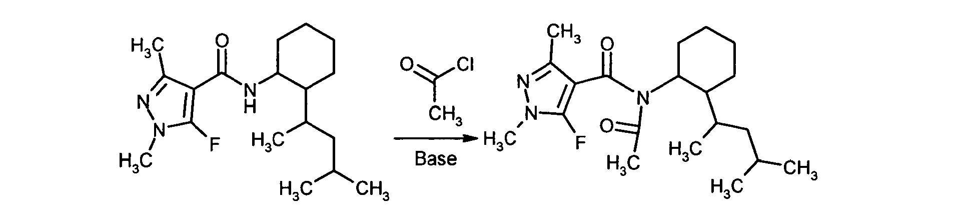

Die zur Durchführung des erfindungsgemäßen Verfahrens (b) als Ausgangsstoffe benötigten 2-Alkylcycloalk(en)yl-carboxamide sind durch die Formel (I-a) allgemein definiert. In dieser Formel (I-a) haben X, s, L, R² und A bevorzugt, besonders bevorzugt bzw. ganz besonders bevorzugt diejenigen Bedeutungen, die bereits im Zusammenhang mit der Beschreibung der erfindungsgemäßen Verbindungen der Formel (I) als bevorzugt, besonders bevorzugt bzw. ganz besonders bevorzugt für diese Reste angegeben wurden.

Die Verbindungen der Formel (I-a) sind erfindungsgemäße Verbindungen und können über Verfahren (a) hergestellt werden.

Die zur Durchführung des erfindungsgemäßen Verfahrens (b) weiterhin als Ausgangsstoffe benötigten Halogenide sind durch die Formel (IV) allgemein definiert. In dieser Formel (IV) steht R^{1A} bevorzugt, besonders bevorzugt bzw. ganz besonders bevorzugt für diejenigen Bedeutungen, die bereits oben für die Verbindungen der Formel (I-b) als bevorzugt, besonders bevorzugt bzw. ganz besonders bevorzugt für diesen Rest angegeben wurden. X⁴ steht für Chlor, Brom oder Iod.

Halogenide der Formel (IV) sind bekannt.

### Reaktionsbedingungen

Als Verdünnungsmittel zur Durchführung des erfindungsgemäßen Verfahrens (a) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; deren Gemische mit Wasser oder reines Wasser.

Das erfindungsgemäße Verfahren (a) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Das erfindungsgemäße Verfahren (a) wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester, Chlorameisensäureisopropylester, Chlorameisensäureisobutylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ), Triphenylphosphin/Tetrachlorkohlenstoff oder Bromtripyrrolidinophosphonium-hexafluorophosphat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens (a) in einem größeren Bereich variiert werden. Im Allgemeinen arbeitet man bei Temperaturen von 0°C bis 150°C, vorzugsweise bei Temperaturen von 0°C bis 80°C.

Zur Durchführung des erfindungsgemäßen Verfahrens (a) zur Herstellung der Verbindungen der Formel (I) setzt man pro Mol des Carbonsäure-Derivates der Formel (II) im Allgemeinen 0,8 bis 15 Mol, vorzugsweise 0,8 bis 8 Mol an Anilin-Derivat der Formel (III) ein.

Als Verdünnungsmittel zur Durchführung des erfindungsgemäßen Verfahrens (b) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid.

Das erfindungsgemäße Verfahren (b) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens (b) zur Herstellung der Verbindungen der Formel (I) setzt man pro Mol des 2-Alkyl-cycloalk(en)yl-carboxamide der Formel (I-a) im Allgemeinen 0,2 bis 5 Mol, vorzugsweise 0,5 bis 2 Mol an Halogenid der Formel (IV) ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Als Verdünnungsmittel zur Durchführung des erfindungsgemäßen Verfahrens (c) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid, Alkohole, wie z.B. Methanol, Ethanol, iso-Propanol, n-, sec, oder tert. Butanol, deren Gemische mit Wasser oder reines Wasser.

Das erfindungsgemäße Verfahren (c) wird in Gegenwart einer Base durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie z.B. Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen von -20°C bis 150°C, vorzugsweise bei Temperaturen von 0°C bis 110°C.

Zur Durchführung des erfindungsgemäßen Verfahrens (c) zur Herstellung der Verbindungen der Formel (III) setzt man pro Mol des cyclischen Ketons der Formel (V) im Allgemeinen 0,2 bis 5 Mol, vorzugsweise 0,5 bis 2 Mol an Carbonyl-Verbindung der Formel (VI) ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

### Verfahren c-2 reduktive Aminierung

Als Verdünnungsmittel zur Durchführung des erfindungsgemäßen Verfahrens (c-2) kommen alle inerten organischen Lösungsmittel in Betracht. Hierzu gehören vorzugsweise aliphatische, alicyclische oder aromatische Kohlenwasserstoffe, wie z.B. Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlormethan, Dichlorethan oder Trichlorethan; Ether, wie Diethylether, Diisopropylether, Methyl-tert-butylether, Methyl-tert-Amylether, Dioxan, Tetrahydrofuran, 1,2- Dimethoxyethan, 1,2-Diethoxyethan oder Anisol oder Amide, wie N,N-Dimethylformamid, N,N-Dimethylacetamid, N-Methylformanilid, N-Methylpyrrolidon oder Hexamethylphosphorsäuretriamid, Alkohole, wie z.B. Methanol, Ethanol, iso-Propanol, n-, sec, oder tert. Butanol, deren Gemische mit Wasser oder reines Wasser.

Die reduktive Aminierung im erfindungsgemäßen Verfahren (c) wird in Gegenwart eines Amins und eines Reduktionsmittels durchgeführt. Als Aminkomponente kommen Ammoniak, Ammoniaksalze, wie z.B. Ammoniumformiat, aber auch einfach substituiertes Ammoniak, wie. z.B. Methylamin, Ethylamin, Propylamin, Cyclopropylamin etc. infrage. Als Reduktionsmittel kommen alle üblichen anorganischen oder organischen Reduktionsmittel infrage. Hierzu gehören vorzugsweise Erdalkalimetall- oder Alkalimetallhydride, oder Borhydride, wie z.B. Natriumhydrid, Natriumcyanoborhydrid, Natriumborhydrid oder Wasserstoffquellen, wie z.B. elementarer Wasserstoff, Hydrazin, Cyclohexandien oder Formiate oder auch die Formiate der entsprechenden Aminkomponenten.

Die reduktive Aminierung im erfindungsgemäßen Verfahren (c) wird gegebenenfalls in Gegenwart eines Katalysators durchgeführt. Als Katalysator kommen handelsübliche Katalysatoren, wie z. B. Hydrierungskatalysatoren, z. B. elementares Pd, Ni (oder auch Raney-Nickel), Pt, Fe, Ru, Os oder deren Salze infrage. Diese Katalysatoren können auf Trägermaterialien, wie z. B. Kohle, Silica, Zeolithe etc. aufgebracht oder durch Liganden stabilisiert sein.

Die Reaktionstemperaturen können bei der Durchführung der reduktiven Aminierung im erfmdungsgemäßen Verfahren (c) in einem größeren Bereich variiert werden. Im Allgemeinen arbeitet man bei Temperaturen von 0°C bis 150°C, vorzugsweise bei Temperaturen von 20°C bis 110°C.

Die reduktive Aminierung im erfmdungsgemäßen Verfahren (c) zur Herstellung der Verbindungen der Formel (III) setzt man pro Mol des cyclischen Ketons der Formel (V) im Allgemeinen 0,2 bis 50 Mol, vorzugsweise 1 bis 20 Mol an Amin und Reduktionsmittel, sowie 0,01-10 mol% Katalysator ein.

Wenn nicht anders angegeben, werden alle erfindungsgemäßen Verfahren im Allgemeinen unter Normaldruck durchgeführt. Es ist jedoch auch möglich, unter erhöhtem oder vermindertem Druck - im Allgemeinen zwischen 0,1 bar und 100 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;
Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;
Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;
Blattfleckenkrankheiten und Blattwelken, hervorgerufen durch z.B.
Alternaria-Arten, wie beispielsweise Altemaria solani;
Cercospora-Arten, wie beispielsweise Cercospora beticola;
Cladiosporum-Arten, wie beispielsweise Cladiosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerelle graminicola;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incamata;
Venturia-Arten, wie beispielsweise Venturia inaequalis;
Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.;
Aspergillus-Arten, wie beispielsweise Aspergillus flavus;
Cladosporium-Arten, wie beispielsweise Cladosporium spp.;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;
Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;
Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B. Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizoctonia-Arten, wie beispielsweise Rhizoctonia solani;
Sclerotium-Arten, wie beispielsweise Sclerotium rolfsii;
Krebserkrankungen, Gallen und Hexenbesen, hervorgerufen durch z.B.
Nectria-Arten, wie beispielsweise Nectria galligena;
Welkeerkrankungen hervorgerufen durch z.B.
Monilinia-Arten, wie beispielsweise Monilinia laxa;
Deformationen von Blättern, Blüten und Früchten, hervorgerufen durch z.B.
Taphrina-Arten, wie beispielsweise Taphrina deformans;
Degenerationserkrankungen holziger pflanzen, hervorgerufen durch z.B.
Esca-Arten, wie beispielsweise Phaemoniella clamydospora;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;

Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)

Pilzkrankheiten an Wurzeln und der Stängelbasis verursacht durch z.B.
Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.
Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:

Fungizide:
1) Inhibitoren der Nukleinsäuresynthese: z.B. Benalaxyl, Benalaxyl-M, Bupirimate, Clozylacon, Dimethirimol, Ethirimol, Furalaxyl, Hymexazol, Mefenoxam, Metalaxyl, Metalaxyl-M, Ofurace, Oxadixyl, Oxolinic acid;
2) Inhibitoren von Mitose und Zellteilung: z.B. Benomyl, Carbendazim, Diethofencarb, Ethaboxam, Fuberidazole, Pencycuron, Thiabendazole, Thiophanate-methyl, Zoxamide;
3) Inhibitoren der Respiration (Atmungsketten-Inhibitoren):
3.1) Inhibitoren am Komplex I der Atmungskette: z.B. Diflumetorim;
3.2) Inhibitoren am Komplex II der Atmungskette: z.B. Boscalid/Nicobifen, Carboxin, Fenfuram, Flutolanil, Furametpyr, Furmecyclox, Mepronil, Oxycarboxin, Penthiopyrad, Thifluzamide;
3.3) Inhibitoren am Komplex III der Atmungskette: z.B. Amisulbrom, Azoxystrobin, Cyazofamid, Dimoxystrobin, Enestrobin, Famoxadone, Fenamidone, Fluoxastrobin, Kresoxim-methyl, Metominostrobin, Orysastrobin, Picoxystrobin, Pyraclostrobin, Trifloxystrobin;
4) Entkoppler: z.B. Dinocap, Fluazinam, Meptyldinocap;
5) Inhibotoren der ATP Produktion: z.B. Fentin acetate, Fentin chloride, Fentin hydroxide, Silthiofam;
6) Inhibitoren der Aminosäure- und Protein-Biosynthese: z.B. Andoprim, Blasticidin-S, Cyprodinil, Kasugamycin, Kasugamycin hydrochloride hydrate, Mepanipyrim, Pyrimethanil;
7) Inhibitoren der Signaltransduktion: z.B. Fenpiclonil, Fludioxonil, Quinoxyfen;
8) Inhibitoren der Lipid- und Membran-Synthese: z.B. Biphenyl, Chlozolinate, Edifenphos, Iodocarb, Iprobenfos, Iprodione, Isoprothiolane, Procymidone, Propamocarb, Propamocarb hydrochloride, Pyrazophos, Tolclofos-methyl, Vinclozolin;
9) Inhibitoren der Ergosterol-Biosynthese: z.B. Aldimorph, Azaconazole, Bitertanol, Bromuconazole, Cyproconazole, Diclobutrazole, Difenoconazole, Diniconazole, Diniconazole-M, Dodemorph, Dodemorph acetate, Epoxiconazole, Etaconazole, Fenarimol, Fenbuconazole, Fenhexamid, Fenpropidin, Fenpropimorph, Fluquinconazole, Flurprimidol, Flusilazole, Flutriafol, Furconazole, Furconazole-cis, Hexaconazole, Imazalil, Imazalil sulfate, Imibenconazole, Ipconazole, Metconazole, Myclobutanil, Naftifine, Nuarimol, Oxpoconazole, Paclobutrazol, Pefurazoate, Penconazole, Prochloraz, Propiconazole, Prothioconazole, Pyributicarb, Pyrifenox, Simeconazole, Spiroxamine, Tebuconazole, Terbinafine, Tetraconazole, Triadimefon, Triadimenol, Tridemorph, Triflumizole, Triforine, Triticonazole, Uniconazole, Viniconazole, Voriconazole;
10) Inhibitoren der Zellwandsynthese: z.B. Benthiavalicarb, Dimethomorph, Flumorph, Iprovalicarb, Polyoxins, Polyoxorim, Validamycin A;
11) Inhibitoren der Melanin-Biosynthese: z.B. Carpropamid, Diclocymet, Fenoxanil, Phthalide, Pyroquilon, Tricyclazole;
12) Resistenzinduktoren: z.B. Acibenzolar-S-methyl, Probenazole, Tiadinil;
13) Verbindungen mit Multisite-Aktivität: z.B. Bordeaux Mixture, Captafol, Captan, Chlorothalonil, Copper naphthenate, Copper oxide, Copper oxychloride, Kupferzubereitungen wie z.B. Kupferhydroxid, Kupfersulfat, Dichlofluanid, Dithianon, Dodine, Dodine free base, Ferbam, Fluorofolpet, Folpet, Guazatine, Guazatine acetate, Iminoctadine, Iminoctadine albesilate, Iminoctadine triacetate, Mancopper, Mancozeb, Maneb, Metiram, Metiram Zinc, Oxine-Copper, Propineb, Sulphur und Schweferlzubereitungen wie z.B. Calcium polysulphide, Thiram, Tolylfluanid, Zineb, Ziram;
14) eine Verbindung aus der folgenden Liste: (2E)-2-(2-{[6-(3-Chlor-2-methylphenoxy)-5-fluorpyrimidin-4-yl]oxy}phenyl)-2-(methoxyimino)-N-methylacetamid, (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, 1-(4-Chlorphenyl)-2-(1H-1,2,4-triazol-1-yl)cycloheptanol, 1-[(4-Methoxyphenoxy)methyl]-2,2-dimethylpropyl-1H-imidazol-1-carboxylat, 2-(4-Chlorphenyl)-N-{2-[3-methoxy-4-(prop-2-yn-1-yloxy)phenyl]ethyl}-2-(prop-2-yn-1-yloxy)acetamid, 2,3,5,6-Tetrachlor-4-(methylsulfonyl)pyridin, 2-Butoxy-6-iod-3-propyl-4H-chromen-4-on, 2-Chlor-N-(1,1,3-trimethyl-2,3-dihydro-1H-inden-4-yl)nicotinamid, 2-Phenylphenol und Salze davon, 3,4,5-Trichlorpyridin-2,6-dicarbonitril, 3,4-Dichlor-N-(2-cyanophenyl)-isothiazol-5-carboxamid, 3-[5-(4-Chlorphenyl)-2,3-dimethylisoxazolidin-3-yl]pyridin, 5-Chlor-6-(2,4,6-tnfluorophenyl)-N-[(1R)-1,2,2-trimethylpropyl][1,2,4]triazolo[1,5-a]pyrimidin-7-amin, 5-Chlor-7-(4-methylpiperidin-1-yl)-6-(2,4,6-trifluorphenyl)[1,2,4]triazolo[1,5-a]pyrimidin, 5-Chlor-N-[(1R)-1,2-dimethylpropyl]-6-(2,4,6-trifluorphenyl)[1,2,4]triazolo[1,5-a]pyrimidin-7-amin, 8-Hydroxyquinolinsulfat, Benthiazole, Bethoxazin, Capsimycin, Carvone, Chinomethionat, Cufraneb, Cyflufenamid, Cymoxanil, Dazomet, Debacarb, Dichlorophen, Diclomezine, Dicloran, Difenzoquat, Difenzoquat Methylsulphate, Diphenylamine, Ferimzone, Flumetover, Fluopicolide, Fluoroimide, Flusulfamide, Fosetyl-Aluminium, Fosetyl-Calcium, Fosetyl-Sodium, Hexachlorobenzene, Irumamycin, Methasulfocarb, Methyl (2-chlor-5-{(1E)-N-[(6-methylpyridin-2-yl)methoxy]ethanimidoyl}benzyl)carbamat, Methyl (2E)-2-{2-[({cyclopropyl[(4-methoxyphenyl)imino]methyl}thio)methyl]phenyl}-3-methoxyacrylat, Methyl 1-(2,2-dimethyl-2,3-dihydro-1H-inden-1-yl)-1H-imidazol-5-carboxylat, Methyl 3-(4-chlorphenyl)-3-{[N-(isopropoxycarbonyl)valyl]amino}propanoat, Methyl isothiocyanate, Metrafenone, Mildiomycin, N-(3',4'-Dichlor-5-fluorbiphenyl-2-yl)-3-(difluormethyl)-1-methyl-1H-pyrazol-4-carboxamid, N-(3-Ethyl-3,5,5-trimethylcyclohexyl)-3-(formylamino)-2-hydroxybenzamid, N-(4-Chlor-2-nitrophenyl)-N-ethyl-4-methylbenzensulfonamid, N-[(5-Brom-3-chlorpyridin-2-yl)methyl]-2,4-dichlomicotinamid, N-[1-(5-Brom-3-chlorpyridin-2-yl)ethyl]-2,4-dichlornicotinamid, N-[1-(5-Brom-3-chlorpyridin-2-yl)ethyl]-2-fluor-4-iodnicotinamid, N-[2-(4-{[3-(4-Chlorphenyl)prop-2-yn-1-yl]oxy}-3-methoxyphenyl)ethyl]-N²-(methylsulfonyl)valinamid, N-{(Z)-[(Cyclopropylmethoxy)imino][6-(difluormethoxy)-2,3-difluorphenyl]methyl}-2-phenylacetamid, N-{2-[3-Chlor-5-(trifluormethyl)pyridin-2-yl]ethyl}-2-(trifluormethyl)benzamid, Natamycin, Nickel Dimethyldithiocarbamate, Nitrothal-isopropyl, O-{1-[(4-Methoxyphenoxy)methyl]-2,2-dimethylpropyl} 1H-Imidazol-1-carbothioat, Octhilinone, Oxamocarb, Oxyfenthiin, Pentachlorophenol und Salze, Phosphorsäure und ihre Salze, Piperalin, Propamocarb Fosetylate, Propanosine-Sodium, Proquinazid, Pyrrolnitrine, Quintozene, Tecloftalam, Tecnazene, Triazoxide, Trichlamide, Zarilamid.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

### 1. Acetylcholinesterase (AChE) Inhibitoren

1.1 Carbamate (z.B. Alanycarb, Aldicarb, Aldoxycarb, Allyxycarb, Aminocarb, Azamethiphos, Bendiocarb, Benfuracarb, Bufencarb, Butacarb, Butocarboxim, Butoxycarboxim, Carbaryl, Carbofuran, Carbosulfan, Chloethocarb, Coumaphos, Cyanofenphos, Cyanophos, Dimetilan, Ethiofencarb, Fenobucarb, Fenothiocarb, Formetanate, Furathiocarb, Isoprocarb, Metam-sodium, Methiocarb, Methomyl, Metolcarb, Oxamyl, Pirimicarb, Promecarb, Propoxur, Thiodicarb, Thiofanox, Triazamate, Trimethacarb, XMC, Xylylcarb) 1.2 Organophosphate (z.B. Acephate, Azamethiphos, Azinphos (-methyl, -ethyl), Bromophos-ethyl, Bromfenvinfos (-methyl), Butathiofos, Cadusafos, Carbophenothion, Chlorethoxyfos, Chlorfenvinphos, Chlormephos, Chlorpyrifos (-methyl/-ethyl), Coumaphos, Cyanofenphos, Cyanophos, Chlorfenvinphos, Demeton-S-methyl, Demeton-S-methylsulphon, Dialifos, Diazinon, Dichlofenthion, Dichlorvos/DDVP, Dicrotophos, Dimethoate, Dimethylvinphos, Dioxabenzofos, Disulfoton, EPN, Ethion, Ethoprophos, Etrimfos, Famphur, Fenamiphos, Fenitrothion, Fensulfothion, Fenthion, Flupyrazofos, Fonofos, Formothion, Fosmethilan, Fosthiazate, Heptenophos, Iodofenphos, Iprobenfos, Isazofos, Isofenphos, Isopropyl O-salicylate, Isoxathion, Malathion, Mecarbam, Methacrifos, Methamidophos, Methidathion, Mevinphos, Monocrotophos, Naled, Omethoate, Oxydemeton-methyl, Parathion (-methyl/-ethyl), Phenthoate, Phorate, Phosalone, Phosmet, Phosphamidon, Phosphocarb, Phoxim, Pirimiphos (-methyl/-ethyl), Profenofos, Propaphos, Propetamphos, Prothiofos, Prothoate, Pyraclofos, Pyridaphenthion, Pyridathion, Quinalphos, Sebufos, Sulfotep, Sulprofos, Tebupirimfos, Temephos, Terbufos, Tetrachlorvinphos, Thiometon, Triazophos, Triclorfon, Vamidothion)

### 2. Natrium-Kanal-Modulatoren /Spannungsabhängige Natrium-Kanal-Blocker

2.1 Pyrethroide (z.B. Acrinathrin, Allethrin (d-cis-trans, d-trans), Beta-Cyfluthrin, Bifenthrin, Bioallethrin, Bioallethrin-S-cyclopentyl-isomer, Bioethanomethrin, Biopermethrin, Bioresmethrin, Chlovaporthrin, Cis-Cypermethrin, Cis-Resmethrin, Cis-Permethrin, Clocythrin, Cycloprothrin, Cyfluthrin, Cyhalothrin, Cypermethrin (alpha-, beta-, theta-, zeta-), Cyphenothrin, DDT, Deltamethrin, Empenthrin (1R-isomer), Esfenvalerate, Etofenprox, Fenfluthrin, Fenpropathrin, Fenpyrithrin, Fenvalerate, Flubrocythrinate, Flucythrinate, Flufenprox, Flumethrin, Fluvalinate, Fubfenprox, Gamma-Cyhalothrin, Imiprothrin, Kadethrin, Lambda-Cyhalothrin, Metofluthrin, Permethrin (cis-, trans-), Phenothrin (1R-trans isomer), Prallethrin, Profluthrin, Protrifenbute, Pyresmethrin, Resmethrin, RU 15525, Silafluofen, Tau-Fluvalinate, Tefluthrin, Terallethrin, Tetramethrin (1R-isomer), Tralomethrin, Transfluthrin, ZXI 8901, Pyrethrins (pyrethrum))

2.2 Oxadiazine (z.B. Indoxacarb)

### 3. Acetylcholin-Rezeptor-Agonisten/-Antagonisten

3.1 Chloronicotinyle/Neonicotinoide (z.B. Acetamiprid, Clothianidin, Dinotefuran, Imidacloprid, Nitenpyram, Nithiazine, Thiacloprid, Thiamethoxam)

3.2 Nicotine, Bensultap, Cartap

### 4. Acetylcholin-Rezeptor-Modulatoren

4.1 Spinosyne (z.B. Spinosad)

### 5. GABA-gesteuerte Chlorid-Kanal-Antagonisten

5.1 Cyclodiene Organochlorine (z.B. Camphechlor, Chlordane, Endosulfan, Gamma-HCH, HCH, Heptachlor, Lindane, Methoxychlor

5.2 Fiprole (z.B. Acetoprole, Ethiprole, Fipronil, Vaniliprole)

### 6. Chlorid-Kanal-Aktivatoren

6.1 Mectine (z.B. Abamectin, Avermectin, Emamectin, Emamectin-benzoate, Ivermectin, Milbemectin, Milbemycin)

### 7. Juvenilhormon-Mimetika (z.B. Diofenolan, Epofenonane, Fenoxycarb, Hydroprene, Kinoprene, Methoprene, Pyriproxifen, Triprene)

### 8. Ecdysonagonisten/disruptoren

8.1 Diacylhydrazine (z.B. Chromafenozide, Halofenozide, Methoxyfenozide, Tebufenozide)

### 9. Inhibitoren der Chitinbiosynthese

9.1 Benzoylharnstoffe (z.B. Bistrifluron, Chlofluazuron, Diflubenzuron, Fluazuron, Flucycloxuron, Flufenoxuron, Hexaflumuron, Lufenuron, Novaluron, Noviflumuron, Penfluron, Teflubenzuron, Triflumuron)

9.2 Buprofezin

9.3 Cyromazine

### 10. Inhibitoren der oxidativen Phosphorylierung, ATP-Disruptoren

10.1 Diafenthiuron

10.2 Organotine (z.B. Azocyclotin, Cyhexatin, Fenbutatin-oxide)

### 11. Entkoppler der oxidativen Phoshorylierung durch Unterbrechung des H-Protongradienten

11.1 Pyrrole (z.B. Chlorfenapyr)

11.2 Dinitrophenole (z.B. Binapacyrl, Dinobuton, Dinocap, DNOC)

### 12. Seite-I-Elektronentransportinhibitoren

12.1 METI's (z.B. Fenazaquin, Fenpyroximate, Pyrimidifen, Pyridaben, Tebufenpyrad, Tolfenpyrad)

12.2 Hydramethylnone

12.3 Dicofol

### 13. Seite-II-Elektronentransportinhibitoren

13.1 Rotenone

### 14. Seite-III-Elektronentransportinhibitoren

14.1 Acequinocyl, Fluacrypyrim

### 15. Mikrobielle Disruptoren der Insektendarmmembran

Bacillus thuringiensis-Stämme

### 16. Inhibitoren der Fettsynthese

16.1 Tetronsäuren (z.B. Spirodiclofen, Spiromesifen)

16.2 Tetramsäuren [z.B. 3-(2,5-Dimethylphenyl)-8-methoxy-2-oxo-1-azaspiro[4.5]dec-3-en-4-yl ethyl carbonate (alias: Carbonic acid, 3-(2,5-dimethylphenyl)-8-methoxy-2-oxo-1-azaspiro[4.5]dec-3-en-4-yl ethyl ester, CAS-Reg.-No.: 382608-10-8) and Carbonic acid, cis-3-(2,5-dimethylphenyl)-8-methoxy-2-oxo-1-azaspiro[4.5]dec-3-en-4-yl ethyl ester (CAS-Reg.-No.: 203313-25-1)]

### 17. Carboxamide (z.B. Flonicamid)

### 18. Oktopaminerge Agonisten (z.B. Amitraz)

### 19. Inhibitoren der Magnesium-stimulierten ATPase (z.B. Propargite)

### 20. Phthalamide (z.B. N²-[1,1-Dimethyl-2-(methylsulfonyl)ethyl]-3-iod-N¹-[2-methyl-4-[1,2,2,2-tetrafluor-1-(trifluormethyl)ethyl]phenyl]-1,2-benzenedicarboxamide (CAS-Reg.-No.: 272451-65-7), Flubendiamide)

### 21. Nereistoxin-Analoge

### (z.B. Thiocyclam hydrogen oxalate, Thiosultap-sodium)

### 22. Biologika, Hormone oder Pheromone

### (z.B. Azadirachtin, Bacillus spec., Beauveria spec., Codlemone, Metarrhizium spec., Paecilomyces spec., Thuringiensin, Verticillium spec.)

### 23. Wirkstoffe mit unbekannten oder nicht spezifischen Wirkmechanismen

23.1 Begasungsmittel (z.B. Aluminium phosphide, Methyl bromide, Sulfuryl fluoride)

23.2 Selektive Fraßhemmer (z.B. Cryolite, Flonicamid, Pymetrozine)

23.3 Milbenwachstumsinhibitoren (z.B. Clofentezine, Etoxazole, Hexythiazox)

23.4 Amidoflumet, Benclothiaz, Benzoximate, Bifenazate, Bromopropylate, Buprofezin, Chinomethionat, Chlordimeform, Chlorobenzilate, Chloropicrin, Clothiazoben, Cycloprene, Cyflumetofen, Dicyclanil, Fenoxacrim, Fentrifanil, Flubenzimine, Flufenerim, Flutenzin, Gossyplure, Hydramethylnone, Japonilure, Metoxadiazone, Petroleum, Piperonyl butoxide, Potassium oleate, Pyrafluprole, Pyridalyl, Pyriprole, Sulfluramid, Tetradifon, Tetrasul, Triarathene, Verbutin, ferner die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO 96/37494, WO 98/25923), sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, weiter entwickeltes Wurzelsystem, höhere Beständigkeit der Pflanzenart bzw. Pflanzensorte, gesteigertes Wachstum der Schösslinge, höhere Pflanzenvitalität, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, größere Früchte, höhere Pflanzengröße, grünere Blattfarbe, frühere Blüte, höhere Qualität und/oder höherer Ernährungswert der Ernteprodukte, höhere Zuckerkonzentration in den Früchten, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfmdungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Offenbart sind 2-Alkyl-cycloalk(en)yl-carboxamide der Formel (I) 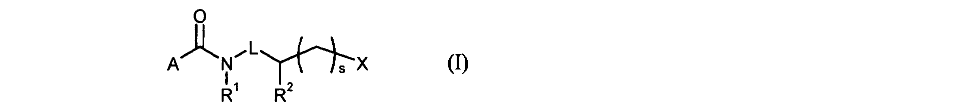 in welcher
- X: für -CR³R⁴R⁵ oder -SiR⁴⁹R⁵⁰R⁵¹ steht
- s: für 1 oder 2 steht,
- R¹: für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁-C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰ steht,
- L: für L¹ oder L² steht,
- L¹: für gegebenenfalls einfach bis vierfach, gleich oder verschieden durch Fluor, Chlor, C₁-C₄-Alkyl oder C₁-C₄-Haloalkyl substituiertes C₃-C₇-Cycloalkyl-1,2-en (C₃-C₇-Cycloalkyl-1,2-diyl) steht,
- L²: für gegebenenfalls einfach bis vierfach, gleich oder verschieden durch Fluor, Chlor, C₁-C₄-Alkyl oder C₁-C₄-Haloalkyl substituiertes Cyclohexenylen (Cyclohexendiyl) steht,
- R²: für Wasserstoff, Halogen, C₁-C₄-Alkyl oder C₁-C₄-Halogenalkyl mit 1 bis 9 Fluor-, Chlor- und/oder Bromatomen steht,
- R³: für Halogen, C₁-C₈-Alkyl oder C₁-C₆-Halogenalkyl mit 1 bis 13 Fluor-, Chlor-und/oder Bromatomen steht,
- R⁴: für Halogen, C₁-C₈-Alkyl oder C₁-C₆-Halogenalkyl mit 1 bis 13 Fluor-, Chlor-und/oder Bromatomen steht,
- R⁵: für Wasserstoff, Halogen, C₁-C₈-Alkyl oder C₁-C₆-Halogenalkyl mit 1 bis 13 Fluor-, Chlor- und/oder Bromatomen steht,
- R³ und R⁴: außerdem gemeinsam mit dem Kohlenstoffatom, an welches sie gebunden sind, einen 3- bis 6-gliedrigen carbocyclischen oder heterocyclischen, gesättigten oder ungesättigten gegebenenfalls durch Halogen, C₁-C₄-Alkyl, C₁-C₄-Alkoxy, C₁-C₄-Haloalkyl oder C₁-C₄-Haloalkoxy substituierten Ring bilden,
- R⁴⁹ und R⁵⁰: unabhängig voneinander für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl oder C₁-C₆-Halogenalkyl stehen,
- R⁵¹: für Wasserstoff, C₁-C₈-Alkyl, C₁-C₈-Alkoxy, C₁-C₄-Alkoxy-C₁-C₄-alkyl, C₁-C₄-Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,
- R⁶: für Wasserstoff, C₁-C₈-Alkyl, C₁-C₈-Alkoxy, C₁-C₄-Alkoxy-C₁-C₄-alkyl, C₃-C₈-Cycloalkyl; C₁-C₆-Halogenalkyl, C₁-C₆-Halogenalkoxy, Halogen-C₁-C₄-alkoxy-C₁-C₄-alkyl, C₃-C₈-Halogencycloalkyl mit jeweils 1 bis 9 Fluor-, Chlor- und/oder Bromatomen steht,
- R⁷ und R⁸: unabhängig voneinander jeweils für Wasserstoff, C₁-C₈-Alkyl, C₁-C₄-Alkoxy-C₁-C₄-alkyl, C₃-C₈-Cycloalkyl; C₁-C₈-Halogenalkyl, Halogen-C₁-C₄-alkoxy-C₁-C₄-alkyl, C₃-C₈-Halogencycloalkyl mit jeweils 1 bis 9 Fluor-, Chlor- und/oder Bromatomen stehen,
- R⁷ und R⁸: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹¹ enthalten kann,
- R⁹ und R¹⁰: unabhängig voneinander für Wasserstoff, C₁-C₈-Alkyl, C₃-C₈-Cycloalkyl; C₁-C₈-Halogenalkyl, C₃-C₈-Halogencycloalkyl mit jeweils 1 bis 9 Fluor-, Chlor- und/oder Bromatomen stehen,
- R⁹ und R¹⁰: außerdem gemeinsam mit dem Stickstoffatom, an das sie gebunden sind, einen gegebenenfalls einfach oder mehrfach, gleich oder verschieden durch Halogen oder C₁-C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹¹ enthalten kann,
- R¹¹: für Wasserstoff oder C₁-C₆-Alkyl steht,
- A: für den Rest der Formel (A1) 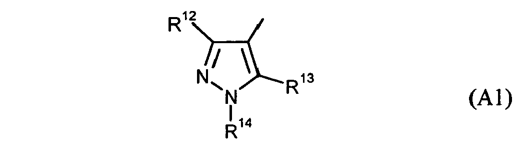 steht, in welcher
- R¹²: für Wasserstoff, Cyano, Halogen, Nitro, C₁-C₄-Alkyl, C₁-C₄-Alkoxy, C₁-C₄-Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
- R¹³: für Wasserstoff, Halogen, Cyano, C₁-C₄-Alkyl, C₁-C₄-Alkoxy oder C₁-C₄-Alkylthio steht,
- R¹⁴: für Wasserstoff, C₁-C₄-Alkyl, Hydroxy-C₁-C₄-alkyl, C₂-C₆-Alkenyl, C₃-C₆-Cycloalkyl, C₁-C₄-Alkylthio-C₁-C₄-alkyl, C₁-C₄-Alkoxy-C₁-C₄-alkyl, C₁-C₄-Halogenalkyl, C₁-C₄-Halogenalkylthio-C₁-C₄-alkyl, C₁-C₄-Halogenalkoxy-C₁-C₄-alkyl mit jeweils 1 bis 5 Halogenatomen, oder Phenyl steht,
- oder:
- A: für den Rest der Formel (A2) 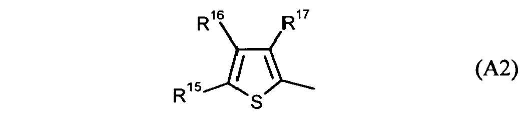 steht, in welcher
- R¹⁵ und R¹⁶: unabhängig voneinander für Wasserstoff, Halogen, C₁-C₄-Alkyl oder C₁-C₄-Halogenalkyl mit 1 bis 5 Halogenatomen steht,
- R¹⁷: für Halogen, Cyano oder C₁-C₄-Alkyl, oder C₁-C₄-Halogenalkyl oder C₁-C₄-Halogenalkoxy mit jeweils 1 bis 5 Halogenatomen steht,
- oder: A für den Rest der Formel (A3) 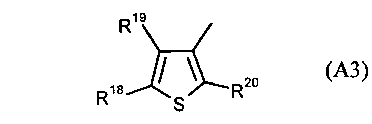 steht, in welcher
- R¹⁸ und R¹⁹: unabhängig voneinander für Wasserstoff, Halogen, C₁-C₄-Alkyl oder C₁ C₄-Halogenalkyl mit 1 bis 5 Halogenatomen steht,
- R²⁰: für Wasserstoff, Halogen, C₁-C₄-Alkyl oder C₁-C₄-Halogenalkyl mit 1 bis 5 Halogenatomen steht,
- oder: A für den Rest der Formel (A4) 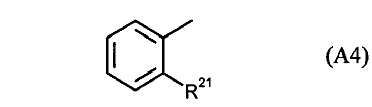 steht, in welcher
- R²¹: für Wasserstoff, Halogen, Hydroxy, Cyano, C₁-C₆-Alkyl, C₁-C₄-Halogenalkyl, C₁-C₄-Halogenalkoxy oder C₁-C₄-Halogenalkylthio mit jeweils 1 bis 5 Halogenatomen steht,
- oder: A für den Rest der Formel (A5) 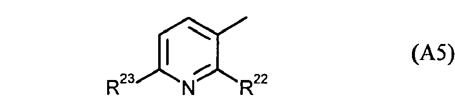 steht, in welcher R²² für Halogen, Hydroxy, Cyano, C₁-C₄-Alkyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht, R²³ für Wasserstoff, Halogen, Cyano, C₁-C₄-Alkyl, C₁-C₄-Alkoxy, C₁-C₄-Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
- oder: A für den Rest der Formel (A6) 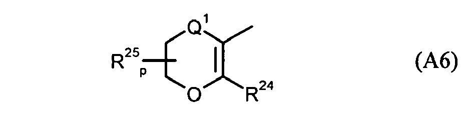 steht, in welcher
- R²⁴: für C₁-C₄-Alkyl oder C₁-C₄-Halogenalkyl mit 1 bis 5 Halogenatomen steht,
- R²⁵: für C₁-C₄-Alkyl steht,
- Q¹: für S (Schwefel), O (Sauerstoff) SO, SO₂ oder CH₂ steht,
- p: für 0, 1 oder 2, wobei R²⁵ für identische oder verschiedene Reste steht, wenn p für 2 steht,
- oder: A für den Rest der Formel (A7) 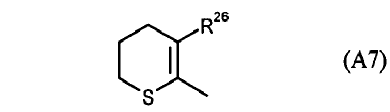 steht, in welcher
- R²⁶: für C₁-C₄-Alkyl oder C₁-C₄-Halogenalkyl mit 1 bis 5 Halogenatomen steht,
- oder: A für den Rest der Formel (A8) 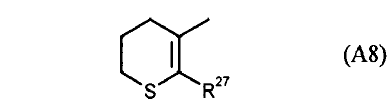 steht, in welcher
- R²⁷: für C₁-C₄-Alkyl oder C₁-C₄-Halogenalkyl mit 1 bis 5 Halogenatomen steht,
- oder: A für den Rest der Formel (A9) 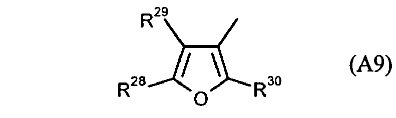 steht, in welcher
- R²⁸ und R²⁹: unabhängig voneinander für Wasserstoff, Halogen, Amino, C₁-C₄-Alkyl oder C₁-C₄-Halogenalkyl mit 1 bis 5 Halogenatomen stehen,
- R³⁰: für Wasserstoff, Halogen, C₁-C₄-Alkyl oder C₁-C₄-Halogenalkyl mit 1 bis 5 Halogenatomen steht,
- oder: A für den Rest der Formel (A 10) 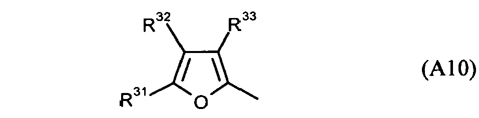 steht, in welcher
- R³¹ und R³²: unabhängig voneinander für Wasserstoff, Halogen, Amino, Nitro, C₁-C₄-Alkyl oder C₁-C₄-Halogenalkyl having 1 bis 5 Halogenatomen stehen,
- R³³: für Wasserstoff, Halogen, C₁-C₄-Alkyl oder C₁-C₄-Halogenalkyl mit 1 bis 5 Halogenatomen steht,
- oder: A für den Rest der Formel (A11) 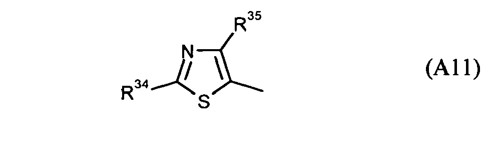 steht, in welcher
- R³⁴: für Wasserstoff, Halogen, Amino, C₁-C₄-Alkylamino, Di-(C₁-C₄ alkyl)amino, Cyano, C₁-C₄-Alkyl oder C₁-C₄-Halogenalkyl mit 1 bis 5 Halogenatomen steht,
- R³⁵: für Halogen, C₁-C₄-Alkyl oder C₁-C₄-Halogenalkyl mit 1 bis 5 Halogenatomen steht,
- oder: A für den Rest der Formel (A12) 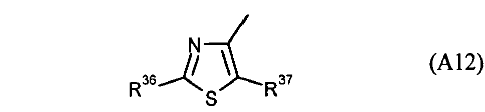 steht, in welcher
- R³⁶: für Wasserstoff, Halogen, Amino, C₁-C₄-Alkylamino, Di-(C₁-C₄ alkyl)amino, Cyano, C₁-C₄-Alkyl oder C₁-C₄-Halogenalkyl mit 1 bis 5 Halogenatomen steht,
- R³⁷: für Halogen, C₁-C₄-Alkyl oder C₁-C₄-Halogenalkyl mit 1 bis 5 Halogenatomen steht,
- oder: A für den Rest der Formel (A13) 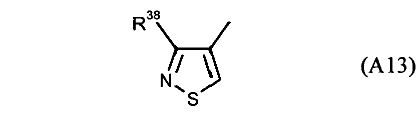 steht, in welcher
- R³⁸: für Halogen, C₁-C₄-Alkyl oder C₁-C₄-Halogenalkyl mit 1 bis 5 Halogenatomen steht,
- oder: A für den Rest der Formel (A14) 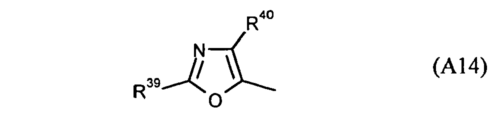 steht, in welcher
- R³⁹: für Wasserstoff oder C₁-C₄-Alkyl steht,
- R⁴⁰: für Halogen oder C₁-C₄-Alkyl steht,
- oder: A für den Rest der Formel (A15) 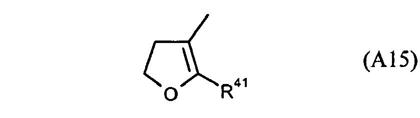 steht, in welcher
- R⁴¹: für C₁-C₄-Alkyl oder C₁-C₄-Halogenalkyl mit 1 bis 5 Halogenatomen steht,
- oder: A für den Rest der Formel (A16) 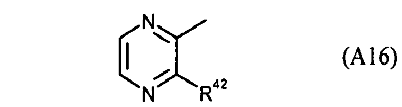 steht, in welcher
- R⁴²: für Wasserstoff, Halogen, C₁-C₄-Alkyl oder C₁-C₄-Halogenalkyl mit 1 bis 5 Halogenatomen steht,
- oder: A für den Rest der Formel (A17) 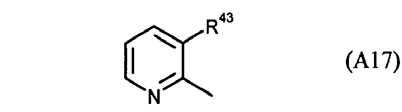 steht, in welcher
- R⁴³: für Halogen, Hydroxy, C₁-C₄-Alkyl, C₁-C₄-Alkoxy, C₁-C₄-Alkylthio, C₁-C₄ Halogenalkyl, C₁-C₄-Halogenalkylthio oder C₁-C₄-Halogenalkoxy mit jeweils 1 bis 5 Halogenatomen steht,
- oder: A für den Rest der Formel (A18) 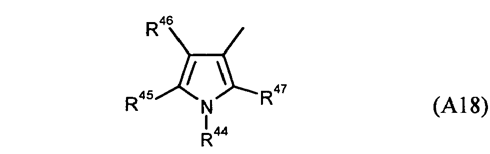 steht, in welcher
- R⁴⁴: für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄ Alkylsulfonyl, Di(C₁-C₄-alkyl)aminosulfonyl, C₁-C₆-Alkylearbonyl oder jeweils gegebenenfalls substituiertes Phenylsulfonyl oder Benzoyl steht,
- R⁴⁵: für Wasserstoff, Halogen, C₁-C₄-Alkyl oder C₁-C₄-Halogenalkyl mit 1 bis 5 Halogenatomen steht,
- R⁴⁶: für Wasserstoff, Halogen, Cyano, C₁-C₄-Alkyl oder C₁-C₄-Halogenalkyl mit 1 bis 5 Halogenatomen steht,
- R⁴⁷: für Wasserstoff, Halogen, C₁-C₄-Alkyl oder C₁-C₄-Halogenalkyl mit 1 bis 5 Halogenatomen steht,
- oder:
- A: für den Rest der Formel (A19) 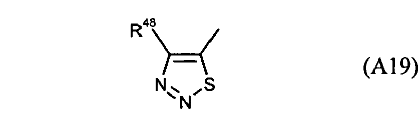 steht, in welcher
- R⁴⁸: für C₁-C₄-Alkyl steht.

Offenbart ist ein Verfahren zum Herstellen der 2-Alkyl-cycloalk(en)yl-carboxamide der Formel (I) wie oben angegeben, dadurch gekennzeichnet, dass man (a) Carbonsäure-Derivate der Formel (II) 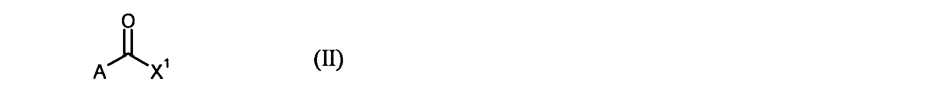 in welcher A die in Anspruch 1 angegebenen Bedeutungen hat und
- X¹: für Halogen oder Hydroxy steht,
mit Anilin-Derivaten der Formel (III) 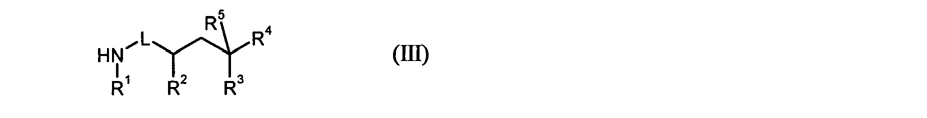 in welcher R¹, L, R², R³, R⁴ und R⁵ die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder (b) 2-Alkyl-cycloalk(en)yl-carboxamide der Formel (I-a) 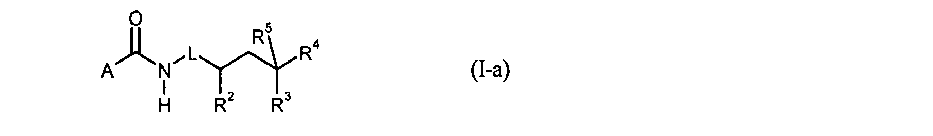 in welcher L, R², R³, R⁴, R⁵ und A die in Anspruch 1 angegebenen Bedeutungen haben, mit Halogeniden der Formel (IV)

R^{1A}-X² (IV)

in welcher
- R^{1A}: für C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)-carbonyl-C₁-C₃-alkyl, (C₁-C₃-Alkoxy)carbonyl-C₁C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)-carbonyl, (C₃-C₈-Cycloalkyl)carbonyl; (C₁-C₆-Halogenalkyl)carbonyl, (C₁-C₆ Halogenalkoxy)carbonyl, (Halogen-C₁-C₄-alkoxy-C₁-C₄-alkyl)carbonyl, (C₃ C₈-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰ steht, R⁶, R⁷, R⁸, R⁹ und R¹⁰ die in Anspruch 1 angegebenen Bedeutungen haben,
- X²: für Chlor, Brom oder Iod steht, in Gegenwart einer Base und in Gegenwart eines Verdünnungsmittels umsetzt.

Offenbart sind Mittel zur Bekämpfung unerwünschter Mikroorganismen, gekennzeichnet durch einen Gehalt an mindestens einem 2-Alkyl-cycloalk(en)yl-carboxamid der Formel (I) wie oben definiert neben Streckmitteln und/oder oberflächenaktiven Stoffen.
Offenbart sind Verwendung von 2-Alkyl-cycloalk(en)yl-carboxamiden der Formel (I) wie oben definiert zur Bekämpfung unerwünschter Mikroorganismen.
Offenbart sind Verfahren zur Bekämpfung unerwünschter Mikroorganismen, dadurch gekennzeichnet, dass man 2-Allcyl-cycloalk(en)yl-carboxamide der Formel (I) wie oben definiert auf die Mikroorganismen und/oder deren Lebensraum ausbringt.
Offenbart sind Verfahren zur Herstellung von Mitteln zur Bekämpfung unerwünschter Mikroorganismen, dadurch gekennzeichnet, dass man 2-Alkyl-cycloalk(en)yl-carboxamide der Formel (I) wie oben definiert mit Streckmitteln und/oder oberflächenaktiven Stoffen vermischt.

Die Herstellung und die Verwendung der erfindungsgemäßen Wirkstoffe geht aus den folgenden Beispielen hervor.

### Herstellungsbeispiele

### Beispiel 1

### Herstellung von 2-(1,3-Dimethylbutyl)cyclohexanamin

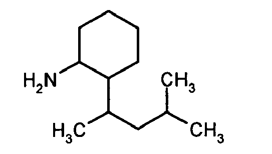

Eine Lösung bestehend aus 20,0 g (0,113 mol) 2-(1,3-Dimethylbutyl)anilin in 300 ml Tetrahydrofuran wird mit 5 g Ru/C 5%ig versetzt und bei 120°C 24 Stunden mit 100 bar Wasserstoff hydriert. Nach Abkühlen auf Raumtemperatur wird der Katalysator über Celite 545 abfiltriert und das Produkt im Vakuum aufkonzentriert. Man erhält 19,1 g (92,4 % der Theorie) an 2-(1,3-Dimethylbutyl)cyclohexanamin mit einem Gehalt von 100 % laut HPLC und einem logP (pH 2,3) von 4,07.

### Herstellung von 4-(Difluormethyl)-N-[2-(1,3-dimethylbutyl)cyclohexyl]-2-methyl-1,3-thiazol-5-carbonsäureamid

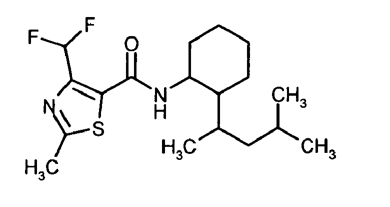

Zu einer Lösung aus 0,16 g (0,82 mmol) 4-(Difluormethyl)-2-methyl-1,3-thiazol-5-carbonsäure und einem Tropfen DMF in 15 ml Dichlormethan werden bei Raumtemperatur 0,104 g (0,82 mmol) Oxalsäuredichlorid getropft und es wird 1 Stunde bei Raumtemperatur gerührt.

Zu einer Lösung bestehend aus 0,15 g (0,82 mmol) 2-(1,3-Dimethylbutyl)cyclohexanamin und 0,25 g (0,0025mol) Triethylamin in 5 ml Dichlormethan wird die oben beschriebene Säurechloridlösung bei Raumtemperatur zugetropft und die Reaktionsmischung 12 Stunden bei Raumtemperatur gerührt.

Man wäscht zweimal mit je 10 ml Wasser, trocknet über Natriumsulfat und engt im Vakuum ein. Der Rückstand wird mit n-Hexan/Methyl-tert-butylether (3:1) an Kieselgel chromatographiert. Man erhält 0,12 g (38,9 % der Theorie) an 4-(Difluormethyl)-N-[2-(1,3-dimethylbutyl)cyclohexyl]-2-methyl-1,3-thiazol-5-carbonsäureamid als Diastereomerengemisch mit einem Gehalt von 95 % laut HPLC und einem logP (pH2,3) von 4,45/4,53.

**Tabelle 1**

| | | | | | | | | |
|---|---|---|---|---|---|---|---|---|
| 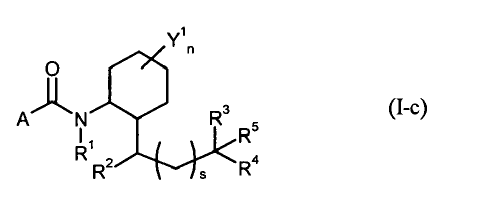 | | | | | | | | |

| Für alle Verbindungen dieser Tabelle 1 ist n = 0. | | | | | | | | |
|---|---|---|---|---|---|---|---|---|
| * die LogP-Werte sind für das Hauptdiastereomer oder, falls detektierbar, für die einzelnen Diastereomeren angegeben: | | | | | | | | |
| **Nr.** | **s** | **R¹** | **R²** | **R³** | **R⁴** | **R⁵** | **A** | **Log P* / Fp. (°C)** |
| I-c-1 | 1 | H | CH₃ | CH₃ | CH₃ | H | 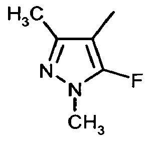 | 4,15 62-65°C |
| I-c-2 | 1 | H | CH₃ | CH₃ | CH₃ | H | 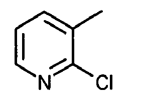 | 4,04 94-98°C |
| I-c-3 | 1 | H | CH₃ | CH₃ | CH₃ | H | 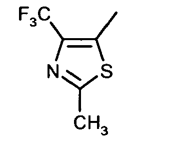 | 4,66/4,71 |
| I-c-4 | 1 | H | CH₃ | CH₃ | CH₃ | H | 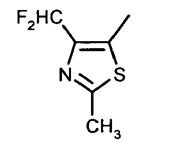 | 4,45/4,53 |
| I-c-5 | 1 | H | CH₃ | CH₃ | CH₃ | H | 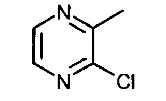 | 4,12/4,23 |
| I-c-6 | 1 | H | CH₃ | CH₃ | CH₃ | H | 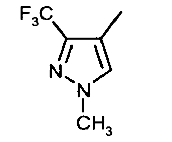 | 4,10/4,53 |
| I-c-7 | 1 | H | CH₃ | CH₃ | CH₃ | H | 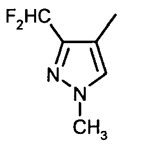 | 3,88/4,28 |
| I-c-8 | 1 | H | CH₃ | CH₃ | CH₃ | H | 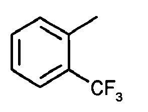 | 4,72/4,88 |
| I-c-9 | 1 | H | CH₃ | CH₃ | CH₃ | H | 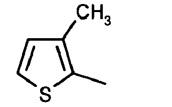 | 4,73/5,15 |
| I-c-10 | 1 | H | CH₃ | CH₃ | CH₃ | H | 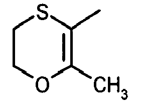 | 4,52/4,83 |
| I-c-11 | 1 | H | CH₃ | CH₃ | CH₃ | H | 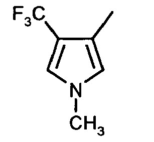 | 4,45/4,83 |
| I-c-12 | 1 | H | CH₃ | CH₃ | CH₃ | H | 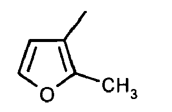 | 4,27/4,58 |
| I-c-13 | 1 | H | CH₃ | CH₃ | CH₃ | H | 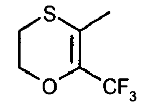 | 4,49/4,67 |
| I-c-14 | 1 | H | CH₃ | CH₃ | CH₃ | H | 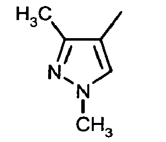 | 3,18/3,47 |
| I-c-15 | 1 | H | CH₃ | CH₃ | CH₃ | H | 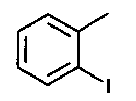 | 4,75/4,92 |
| I-c-16 | 1 | H | CH₃ | CH₃ | CH₃ | H | 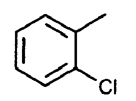 | 4,60/4,83 |
| I-c-17 | 1 | H | CH₃ | CH₃ | CH₃ | H | 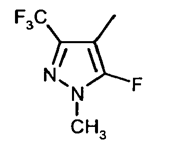 | 4,49/4,75 |
| I-c-18 | 1 | H | CH₃ | CH₃ | CH₃ | H | 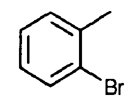 | 4,46/4,86 |
| I-c-19 | 1 | H | H | CH₃ | CH₃ | CH₃ | 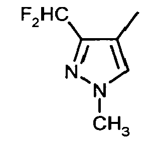 | 4,22 |
| I-c-20 | 1 | H | CH₃ | CH₃ | CH₃ | H | 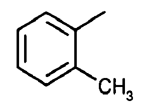 | 4,58/5,00 |
| I-c-21 | 1 | H | CH₃ | CH₃ | CH₃ | H | 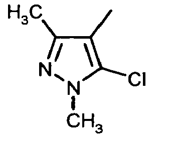 | 4,10/4,43 |
| I-c-22 | 1 | H | CH₃ | CH₃ | CH₃ | H | 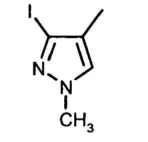 | 3,9/4,32 |
| I-c-23 | 1 | H | H | CH₃ | CH₃ | CH₃ | 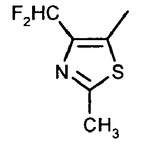 | 4,71 |
| I-c-24 | 1 | H | H | CH₃ | CH₃ | CH₃ | 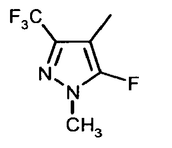 | 4,72 |
| I-c-25 | 1 | H | H | CH₃ | CH₃ | CH₃ | 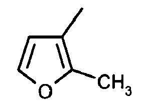 | 4,55 |
| I-c-26 | 1 | H | CH₃ | CH₃ | CH₃ | H | 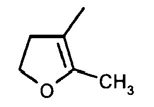 | 4,06 |
| I-c-27 | 1 | H | CH₃ | CH₃ | CH₃ | CH₃ | 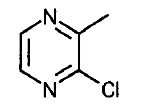 | 4,44/4,64 |
| I-c-28 | 1 | H | CH₃ | CH₃ | CH₃ | CH₃ | 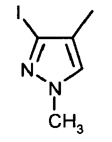 | 4,17/4,33/4,47/4,55 |
| I-c-29 | 1 | H | CH₃ | CH₃ | CH₃ | CH₃ | 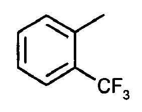 | 4,94/4,98/5,08 |
| I-c-30 | 1 | H | CH₃ | CH₃ | CH₃ | CH₃ | 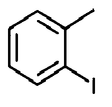 | 5,07/5,22 |
| I-c-31 | 1 | H | CH₃ | CH₃ | CH₃ | CH₃ | 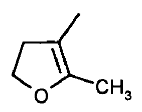 | 4,29/4,37/4,68 |
| I-c-32 | 1 | H | CH₃ | CH₃ | CH₃ | CH₃ | 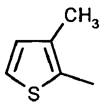 | 4,97/5,07/5,36 |
| I-c-33 | 1 | H | CH₃ | CH₃ | CH₃ | CH₃ | 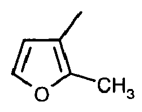 | 4,54/4,63/4,87 |
| I-c-34 | 1 | H | CH₃ | CH₃ | CH₃ | CH₃ | 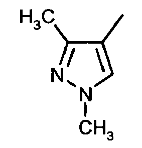 | 3,42/3,54/3,66/3,74 |
| I-c-35 | 1 | H | CH₃ | CH₃ | CH₃ | CH₃ | 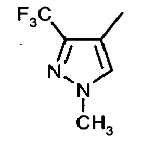 | 4,37/4,48/4,74/4,82 |
| I-c-36 | 1 | H | CH₃ | CH₃ | CH₃ | CH₃ | 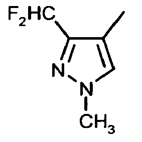 | 4,15/4,27/4,48/4,57 |
| I-c-37 | 1 | H | CH₃ | CH₃ | CH₃ | CH₃ | 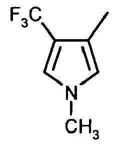 | 4,70/4,80/5,11/5,16 |
| I-c-38 | 1 | H | CH₃ | CH₃ | CH₃ | CH₃ | 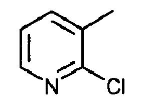 | 4,15/4,21/4,35 |
| I-c-39 | 1 | H | H | CH₃ | CH₃ | CH₃ | 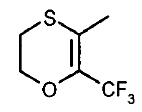 | 4,66 |
| I-c-40 | 1 | H | H | CH₃ | CH₃ | CH₃ | 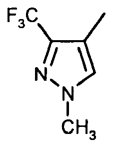 | 4,45 |
| I-c-41 | 1 | H | H | CH₃ | CH₃ | CH₃ | 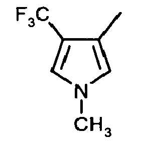 | 4,88 |
| I-c-42 | 1 | H | H | CH₃ | CH₃ | CH₃ | 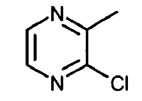 | 4,27 |
| I-c-43 | 1 | H | H | CH₃ | CH₃ | CH₃ | 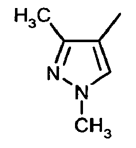 | 3,29/3,42 |
| I-c-44 | 1 | H | CH₃ | CH₃ | CH₃ | CH₃ | 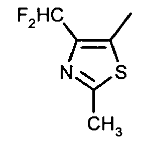 | 4,73/4,83/5,08 |
| I-c-45 | 1 | H | CH₃ | CH₃ | CH₃ | CH₃ | 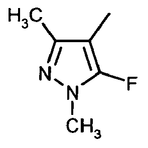 | 4,04/4,19/4,36 |
| I-c-46 | 1 | H | CH₃ | CH₃ | CH₃ | CH₃ | 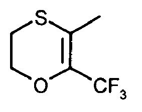 | 4,78/4,86/4,95 |
| I-c-47 | 1 | H | CH₃ | CH₃ | CH₃ | CH₃ | 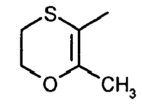 | 4,83/4,90/5,09/5,25 |
| I-c-48 | 1 | H | CH₃ | CH₃ | CH₃ | H | 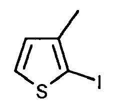 | 4,80/4,85/4,98/5,11 |
| I-c-49 | 1 | H | CH₃ | CH₃ | CH₃ | CH₃ | 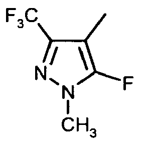 | 4,77/4,85/5,00 |
| I-c-50 | 1 | H | CH₃ | CH₃ | CH₃ | CH₃ | 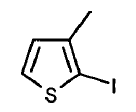 | 5,03/5,11/5,40 |
| I-c-51 | 1 | H | CH₃ | CH₃ | CH₃ | CH₃ | 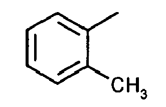 | 4,87/4,94/5,09 |
| I-c-52 | 1 | H | CH₃ | CH₃ | CH₃ | CH₃ | 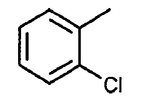 | 4,91/5,11/5,16 |
| I-c-53 | 1 | H | CH₃ | CH₃ | CH₃ | CH₃ | 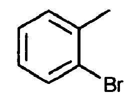 | 4,95/5,12/5,17 |
| I-c-54 | 1 | H | CH₃ | CH₃ | CH₃ | CH₃ | 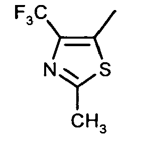 | 4,94/5,00/5,18 |
| I-c-55 | 1 | H | CH₃ | CH₃ | CH₃ | CH₃ | 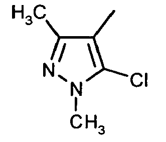 | 4,36/4,52/4,70 |
| I-c-56 | 1 | H | H | CH₃ | CH₃ | H | 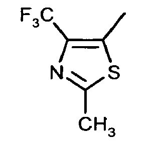 | 4,6 |
| I-c-57 | 1 | H | H | CH₃ | CH₃ | H | 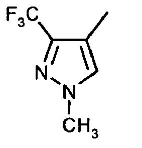 | 4,19 |
| I-c-58 | 1 | H | H | CH₃ | CH₃ | H | 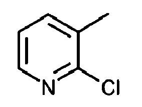 | 3,76 |
| I-c-59 | 1 | H | H | CH₃ | CH₃ | H | 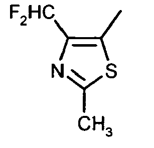 | 4,46 |
| I-c-60 | 1 | H | H | CH₃ | CH₃ | H | 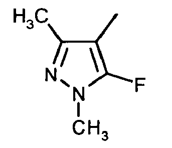 | 3,79 |
| I-c-61 | 1 | H | H | CH₃ | CH₃ | H | 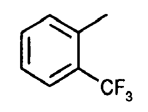 | 4,59 |
| I-c-62 | 1 | H | H | CH₃ | CH₃ | H | 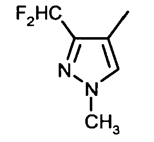 | 3,95 |
| I-c-63 | 1 | H | H | CH₃ | CH₃ | H | 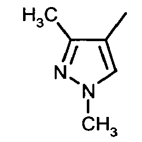 | 3,19 |
| I-c-64 | 1 | H | H | CH₃ | CH₃ | H | 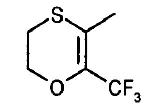 | 4,42 |
| I-c-65 | 1 | H | H | CH₃ | CH₃ | H | 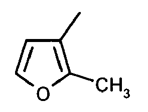 | 4,28 |
| I-c-66 | 1 | H | H | CH₃ | CH₃ | H | 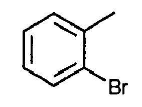 | 4,56 |
| I-c-67 | 1 | H | H | CH₃ | CH₃ | H | 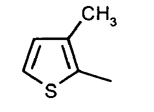 | 4,82 |
| I-c-68 | 1 | H | H | CH₃ | CH₃ | H | 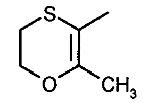 | 4,51 |
| I-c-69 | 1 | H | H | CH₃ | CH₃ | H | 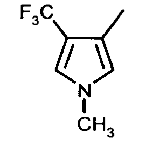 | 4,61 |
| I-c-70 | 1 | H | H | CH₃ | CH₃ | H | 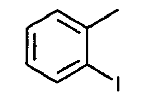 | 4,66 |
| I-c-71 | 1 | H | H | CH₃ | CH₃ | H | 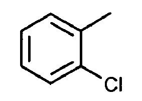 | 4,54 |
| I-c-72 | 1 | H | H | CH₃ | CH₃ | H | 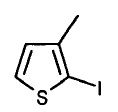 | 4,8 |
| I-c-73 | 1 | H | H | CH₃ | CH₃ | H | 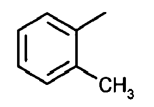 | 4,52 |
| I-c-74 | 1 | H | H | CH₃ | CH₃ | H | 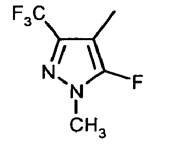 | 4,47 |
| I-c-75 | 2 | H | H | CH₃ | CH₃ | H | 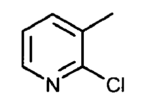 | 4,22 |
| I-c-76 | 2 | H | H | CH₃ | CH₃ | H | 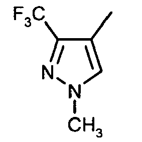 | 4,66 |
| I-c-77 | 1 | H | H | CH₃ | CH₃ | H | 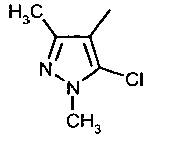 | 4,07 |
| I-c-78 | 2 | H | H | CH₃ | CH₃ | H | 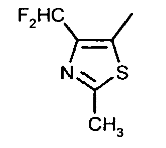 | 4,95 |
| I-c-79 | 2 | H | H | CH₃ | CH₃ | H | 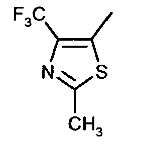 | 5,06 |
| I-c-80 | 1 | H | H | CH₃ | CH₃ | H | 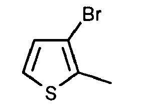 | 5,16/5,47 |
| I-c-81 | 1 | H | H | CH₃ | CH₃ | CH₃ | 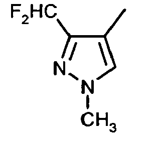 | 4,24 |
| I-c-82 | 1 | H | H | CH₃ | CH₃ | CH₃ | 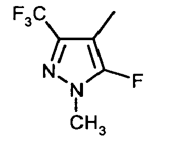 | 4,72 |
| I-c-83 | 2 | H | H | CH₃ | CH₃ | H | 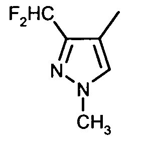 | 4,40 |
| I-c-84 | 2 | H | H | CH₃ | CH₃ | H | 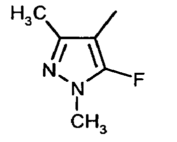 | 4,30 |
| I-c-85 | 2 | H | H | CH₃ | CH₃ | H | 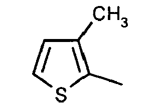 | 5,34 |
| I-c-86 | 2 | H | H | CH₃ | CH₃ | H | 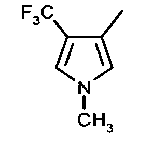 | 5,05 |
| I-c-87 | 2 | H | H | CH₃ | CH₃ | H | 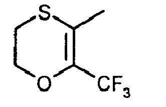 | 4,86 |
| I-c-88 | 1 | H | H | CH₃ | CH₃ | CH₃ | 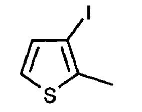 | 5,35 |
| I-c-89 | 1 | H | H | CH₃ | CH₃ | CH₃ | 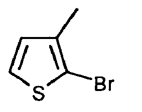 | 4,89 |
| I-c-90 | 1 | H | H | CH₃ | CH₃ | CH₃ | 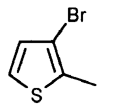 | |
| I-c-91 | 1 | H | H | CH₃ | CH₃ | CH₃ | 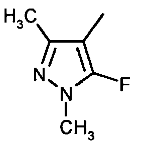 | |
| I-c-92 | 1 | H | H | CH₃ | CH₃ | CH₃ | 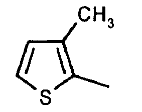 | 4,79 |
| I-c-93 | 1 | H | H | CH₃ | CH₃ | CH₃ | 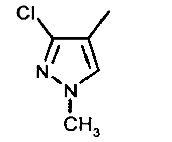 | 4,12/4,47 |
| I-c-94 | 1 | H | H | CH₃ | CH₃ | CH₃ | 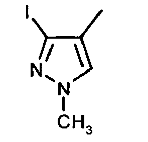 | 4,06/4,28 |
| I-c-95 | 1 | H | H | CH₃ | CH₃ | CH₃ | 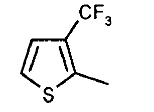 | 5,03 |
| I-c-96 | 1 | H | CH₃ | CH₃ | CH₃ | H | 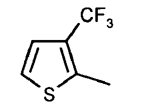 | 5,03 |
| I-c-97 | 1 | H | H | CH₃ | CH₃ | CH₃ | 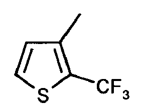 | 4,87 |
| I-c-98 | 1 | H | CH₃ | CH₃ | CH₃ | H | 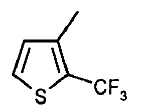 | 4,92 |
| I-c-99 | 1 | H | CH₃ | CH₃ | CH₃ | CH₃ | 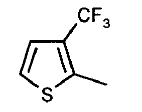 | 5,29 |
| 1-c-100 | 1 | H | H | CH₃ | CH₃ | CH₃ | 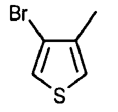 | 4,77 |

**Tabelle 2**

| | | | | | | | | |
|---|---|---|---|---|---|---|---|---|
| 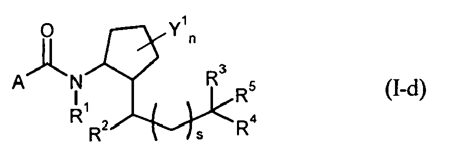 | | | | | | | | |

| Für alle Verbindungen dieser Tabelle 1 ist n = 0. | | | | | | | | |
|---|---|---|---|---|---|---|---|---|
| * die LogP-Werte sind für das Hauptdiastereomer oder, falls detektierbar, für die einzelnen Dia- stereomeren angegeben: | | | | | | | | |
| **Nr.** | **s** | **R¹** | **R²** | **R³** | **R⁴** | **R⁵** | **A** | **Log P* / Fp. (°C)** |
| I-d-1 | 1 | H | H | CH₃ | CH₃ | H | 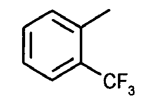 | 4,22/4,53 |

**Tabelle 3**

| | | | | | | | | |
|---|---|---|---|---|---|---|---|---|
| 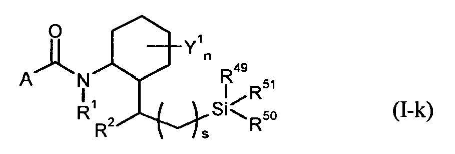 | | | | | | | | |

| Für alle Verbindungen dieser Tabelle 1 ist n = 0. | | | | | | | | |
|---|---|---|---|---|---|---|---|---|
| * die LogP-Werte sind für das Hauptdiastereomer oder, falls detektierbar, für die einzelnen Dia- stereomeren angegeben: | | | | | | | | |
| **Nr.** | **s** | **R¹** | **R²** | **R⁴⁹** | **R⁵⁰** | **R⁵¹** | **A** | **Log P* / Fp. (°C)** |
| I-k-1 | 1 | H | H | CH₃ | CH₃ | CH₃ | 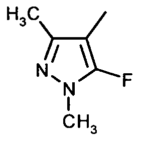 | 4,15 |
| I-k-2 | 1 | H | CH₃ | CH₃ | CH₃ | CH₃ | 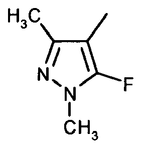 | 4,51 |
| I-k-3 | 1 | H | H | CH₃ | CH₃ | CH₃ | 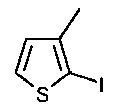 | 5,13 |
| I-k-4 | 1 | H | CH₃ | CH₃ | CH₃ | CH₃ | 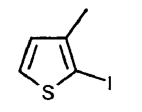 | 5,44 |
| I-k-5 | 1 | H | H | CH₃ | CH₃ | CH₃ | 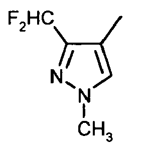 | 4,25/4,54 |
| I-k-6 | 1 | H | CH₃ | CH₃ | CH₃ | CH₃ | 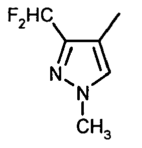 | 4,56 |
| I-k-7 | 1 | H | H | CH₃ | CH₃ | CH₃ | 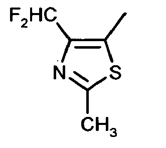 | 4,82/5,06 |
| I-k-8 | 1 | H | CH₃ | CH₃ | CH₃ | CH₃ | 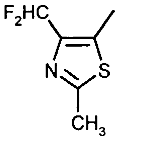 | 5,13 |

Die Bestimmung der angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.

Eluenten für die Bestimmung im sauren Bereich (pH 2,3): 0,1 % wässrige Phosphorsäure, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren LogP-Werte bekannt sind (Bestimmung der LogP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele

### Beispiel A

| **Podosphaera - Test (Apfel) / protektiv** | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension von *Podosphaera leucotricha* inokuliert. Die Pflanzen werden dann im Gewächshaus bei ca. 23°C und einer relativen Luftfeuchtigkeit von ca. 70 % aufgestellt.
10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle A**

| **Podosphaera - Test (Apfel) / protektiv** | | | |
|---|---|---|---|
| Wirkstoff Erfindungsgemäß | | Aufwandmenge an Wirkstoff in g/ha | Wirkungsgrad in % |
| (I-c-4) | 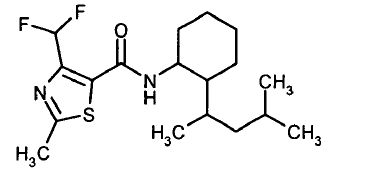 | 100 | 100 |
| (I-c-5) | 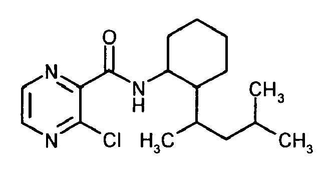 | 100 | 100 |
| (I-c-7) | 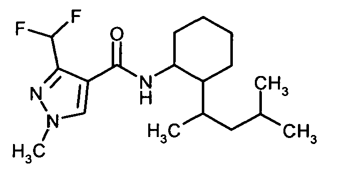 | 100 | 100 |
| (I-c-1) | 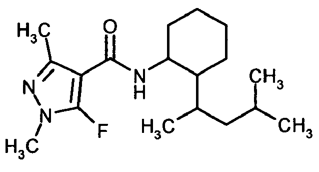 | 100 | 88 |

### Beispiel B

| **Venturia - Test (Apfel) / protektiv** | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers *Venturia inaequalis* inokuliert und verbleiben dann 1 Tag bei ca. 20°C und 100 % relativer Luftfeuchtigkeit in einer Inkubationskabine. Die Pflanzen werden dann im Gewächshaus bei ca. 21 °C und einer relativen Luftfeuchtigkeit von ca. 90 % aufgestellt.
10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle B**

| **Venturia - Test (Apfel) / protektiv** | | | |
|---|---|---|---|
| Wirkstoff Erfindungsgemäß | | Aufwandmenge an Wirkstoff in g/ha | Wirkungsgrad in % |
| (I-c-4) | 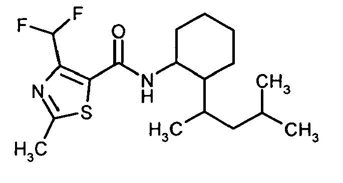 | 100 | 98 |
| (I-c-5) | 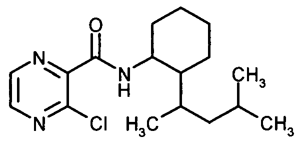 | 100 | 100 |
| (I-c-6) | 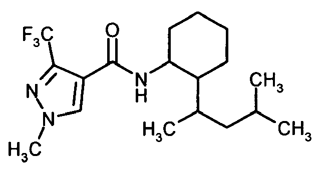 | 100 | 99 |
| (I-c-7) | 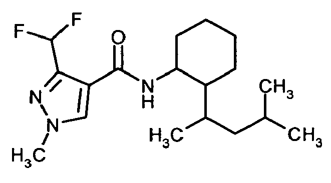 | 100 | 100 |
| (I-c-11) | 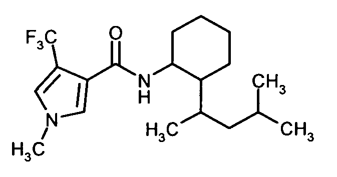 | 100 | 90 |
| (I-c-1) | 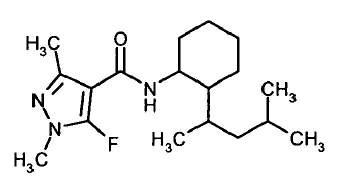 | 100 | 100 |

### Beispiel C

| **Botrytis - Test(Bohne)/protektiv** | | |
|---|---|---|
| Lösungsmittel: | 24,5 | Gewichtsteile Aceton |
| | 24,5 | Gewichtsteile Dimethylacetamid |
| Emulgator: | 1 | Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration. Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit *Botrytis cinerea* bewachsene Agarstückchen aufgelegt. Die inokulierten Pflanzen werden in einer abgedunkelten Kammer bei ca. 20°C und 100 % relativer Luftfeuchtigkeit aufgestellt.
2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

**Tabelle C**

| **Botrytis - Test (Bohne) / protektiv** | | | |
|---|---|---|---|
| Wirkstoff Erfindungsgemäß | | Aufwandmenge an Wirkstoff in g/ha | Wirkungsgrad in % |
| (I-c-4) | 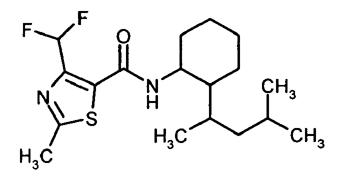 | 500 | 95 |
| (I-c-5) | 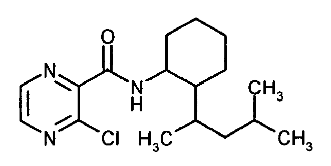 | 500 | 99 |
| (I-c-6) | 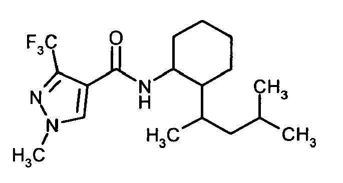 | 500 | 90 |
| (I-c-7) | 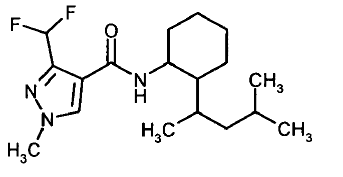 | 500 | 94 |
| (I-c-11) | 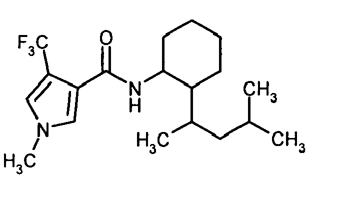 | 500 | 96 |

### Beispiel D

| **Puccinia - Test (Weizen) / protektiv** | | |
|---|---|---|
| Lösungsmittel: | 50 | Gewichtsteile N,N-Dimethylacetamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von *Puccinia recondita* besprüht. Die Pflanzen verbleiben 48 Stunden bei 20°C und 100 % relativer Luftfeuchtigkeit in einer Inkubationskabine. Die Pflanzen werden dann in einem Gewächshaus bei einer Temperatur von ca. 20°C und einer relativen Luftfeuchtigkeit von 80 % aufgestellt.
10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle D**

| **Puccinia - Test (Weizen) / protektiv** | | | |
|---|---|---|---|
| Wirkstoff Erfindungsgemäß | | Aufwandmenge an Wirkstoff in g/ha | Wirkungsgrad in % |
| (I-c-3) | 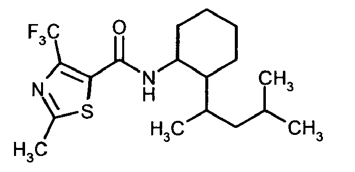 | 500 | 100 |
| (I-c-4) | 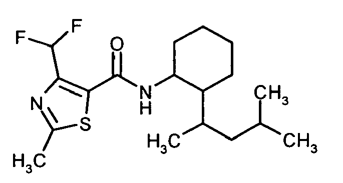 | 500 | 100 |
| (I-c-14) | 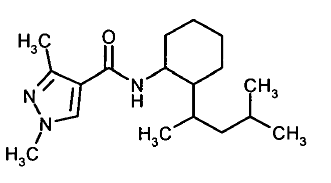 | 500 | 100 |
| (I-c-5) | 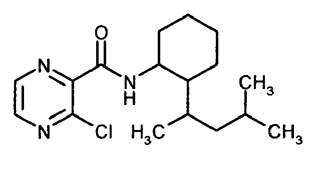 | 500 | 100 |
| (I-c-6) | 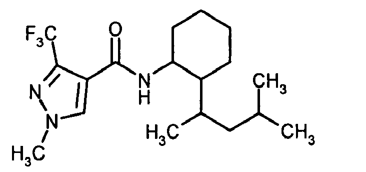 | 500 | 100 |
| (I-c-7) | 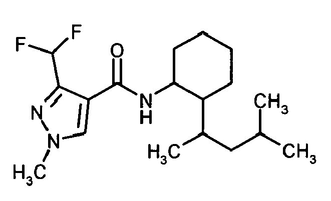 | 500 | 100 |
| (I-c-9) | 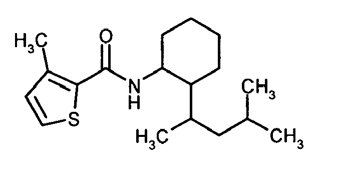 | 500 | 100 |
| (I-c-10) | 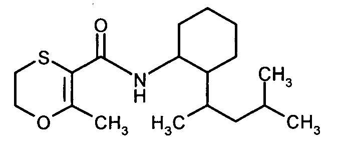 | 500 | 100 |
| (I-c-12) | 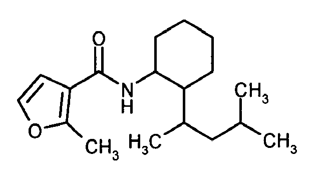 | 500 | 100 |
| (I-c-1) | 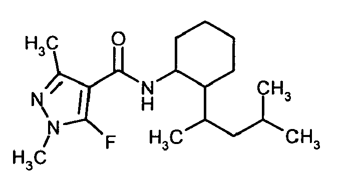 | 500 | 100 |

### Beispiel E

| **Alternaria - Test (Tomate) / protektiv** | | |
|---|---|---|
| Lösungsmittel: | 49 | Gewichtsteile N,N-Dimethylformamid |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von *Alternaria solani* inokuliert und stehen dann 24 h bei 100 % rel. Feuchte und 20°C. Anschließend stehen die Pflanzen bei 96 % rel. Luftfeuchtigkeit und einer Temperatur von 20°C. 7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

**Tabelle E**

| **Alternaria - Test (Tomate) / protektiv** | | | |
|---|---|---|---|
| Wirkstoff Erfindungsgemäß | | Aufwandmenge an Wirkstoff in g/ha | Wirkungsgrad in % |
| (I-c-4) | 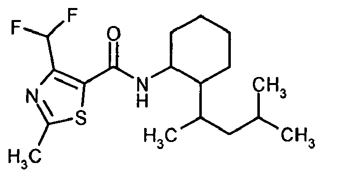 | 750 | 100 |
| (I-c-14) | 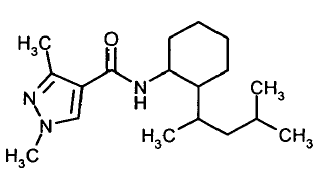 | 750 | 100 |
| (I-c-5) | 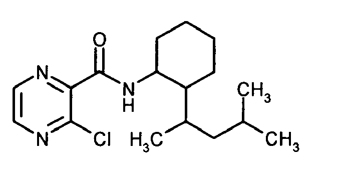 | 750 | 100 |
| (I-c-7) | 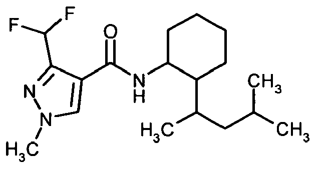 | 750 | 100 |
| (I-c-10) | 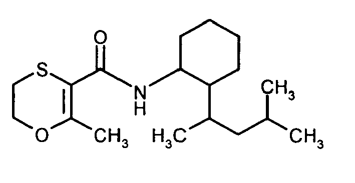 | 750 | 100 |

## Patentansprüche

**1.** 2-Alkyl-cycloalk(en)yl-carboxamide der Formel (I) 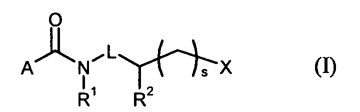 in welcher
X für -CR³R⁴R⁵ oder -SiR⁴⁹R⁵⁰R⁵¹ steht
s für 1 oder 2 steht,
R¹ für Wasserstoff, C₁-C₈-Alkyl, C₁-C₆-Alkylsulfinyl, C₁-C₆-Alkylsulfonyl, C₁-C₄-Alkoxy-C₁-C₄-alkyl, C₃-C₈-Cycloalkyl; C₁-C₆-Halogenalkyl, C₁-C₄-Halogenalkylthio, C₁-C₄-Halogenalkylsulfmyl, C₁-C₄-Halogenalkylsulfonyl, Halogen-C₁-C₄-alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)carbonyl-C₁-C₃-alkyl, (C₁C₃-Alkoxy)carbonyl-C₁-C₃-alkyl; Halogen-(C₁-C₃-alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)carbonyl, (C₃-C₈-Cycloalkyl)carbonyl; (C₁-C₆-Halogenalkyl)carbonyl, (C₁-C₆-Halogenalkoxy)carbonyl, (Halogen-C₁-C₄-alkoxy-C₁-C₄-alkyl)carbonyl, (C₃-C₈-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰ steht,
L für L¹ oder L² steht,
L¹ für eine der folgenden Gruppen 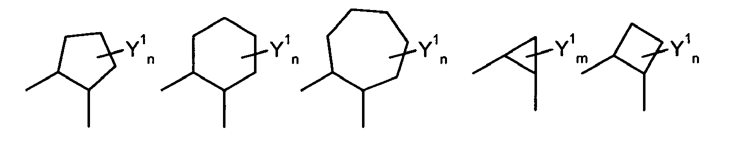 in welchen
m für 0, 1 oder 2 steht,
n für 0, 1, 2, 3, oder 4 steht,
L² für gegebenenfalls einfach bis vierfach, gleich oder verschieden durch Fluor, Chlor, C₁-C₄-Alkyl oder C₁-C₄-Haloalkyl substituiertes Cyclohexenylen (Cyclohexendiyl) steht,
Y¹ für Fluor, Chlor, Methyl, Ethyl, n- oder iso-Propyl, n-, iso-, sec- oder tert-Butyl, Trifluormethyl oder Difluormethyl steht, wobei die Reste Y¹ gleich oder verschieden sein können, wenn n größer als 1 ist,
R² für Wasserstoff, Halogen, C₁-C₄-Alkyl oder C₁-C₄-Halogenalkyl mit 1 bis 9 Fluor-, Chlor- und/oder Bromatomen steht,
R³ für Halogen, C₁-C₈-Alkyl oder C₁-C₆-Halogenalkyl mit 1 bis 13 Fluor-, Chlor und/oder Bromatomen steht,
R⁴ für Halogen, C₁-C₈-Alkyl oder C₁-C₆-Halogenalkyl mit 1 bis 13 Fluor-, Chlor und/oder Bromatomen steht,
R⁵ für Wasserstoff, Halogen, C₁-C₈-Alkyl oder C₁-C₆-Halogenalkyl mit 1 bis 13 Fluor-, Chlor- und/oder Bromatomen steht,
R³ und R⁴ außerdem gemeinsam mit dem Kohlenstoffatom, an welches sie gebunden sind, einen 3- bis 6-gliedrigen carbocyclischen oder heterocyclischen, gesättigten oder ungesättigten gegebenenfalls durch Halogen, C₁-C₄-Alkyl, C₁-C₄-Alkoxy, C₁-C₄ Haloalkyl oder C₁-C₄-Haloalkoxy substituierten Ring bilden,
R⁴⁹ und R⁵⁰ unabhängig voneinander für Wasserstoff, C₁-C₈-Alkyl, C₁-C₈-Alkoxy, C₁-C₄ Alkoxy-C₁-C₄-alkyl, C₁-C₄-Alkylthio-C₁-C₄-alkyl oder C₁-C₆-Halogenalkyl stehen,
R⁵¹ für Wasserstoff, C₁-C₈-Alkyl, C₁-C₈-Alkoxy, C₁-C₄-Alkoxy-C₁-C₄-alkyl, C₁-C₄ Alkylthio-C₁-C₄-alkyl, C₂-C₈-Alkenyl, C₂-C₈-Alkinyl, C₁-C₆-Halogenalkyl, C₂-C₆-Halogenalkenyl, C₂-C₆-Halogenalkinyl, C₃-C₆-Cycloalkyl, oder für jeweils gegebenenfalls substituiertes Phenyl oder Phenylalkyl steht,
R⁶ für Wasserstoff, C₁-C₈-Alkyl, C₁-C₈-Alkoxy, C₁-C₄-Alkoxy-C₁-C₄-alkyl, C₃-C₈ Cycloalkyl; C₁-C₆-Halogenalkyl, C₁-C₆-Halogenalkoxy, Halogen-C₁-C₄-alkoxy-C₁ C₄-alkyl, C₃-C₈-Halogencycloalkyl mit jeweils 1 bis 9 Fluor-, Chlor- und/oder Bromatomen steht,
R⁷ und R⁸ unabhängig voneinander jeweils für Wasserstoff, C₁-C₈-Alkyl, C₁-C₄-Alkoxy-C₁-C₄ alkyl, C₃-C₈-Cycloalkyl; C₁-C₈-Halogenalkyl, Halogen-C₁-C₄-alkoxy-C₁-C₄-alkyl, C₃ C₈-Halogencycloalkyl mit jeweils 1 bis 9 Fluor-, Chlor- und/oder Bromatomen stehen,
R⁷ und R⁸ außerdem gemeinsam mit dem Stickstoffatom, an das sie gebunden sind, einen gegebenenfalls einfach oder mehrfach, gleich oder verschieden durch Halogen oder C₁ C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹¹ enthalten kann,
R⁹ und R¹⁰ unabhängig voneinander für Wasserstoff, C₁-C₈-Alkyl, C₃-C₈-Cycloalkyl; C₁-C₈ Halogenalkyl, C₃-C₈-Halogencycloalkyl mit jeweils 1 bis 9 Fluor-, Chlor- und/oder Bromatomen stehen,
R⁹ und R¹⁰ außerdem gemeinsam mit dem Stickstoffatom, an das sie gebunden sind, einen gegebenenfalls einfach oder mehrfach, gleich oder verschieden durch Halogen oder C₁ C₄-Alkyl substituierten gesättigten Heterocyclus mit 5 bis 8 Ringatomen bilden, wobei der Heterocyclus 1 oder 2 weitere, nicht benachbarte Heteroatome aus der Reihe Sauerstoff, Schwefel oder NR¹¹ enthalten kann,
R¹¹ für Wasserstoff oder C₁-C₆-Alkyl steht,
A für den Rest der Formel (A1) 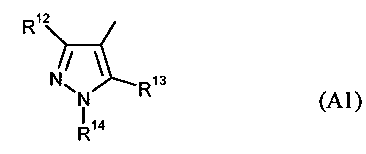 steht, in welcher
R¹² für Wasserstoff, Cyano, Halogen, Nitro, C₁-C₄-Alkyl, C₁-C₄-Alkoxy, C₁-C₄ Alkylthio, C₃-C₆-Cycloalkyl, C₁-C₄-Halogenalkyl, C₁-C₄-Halogenalkoxy oder C₁-C₄-Halogenalkylthio mit jeweils 1 bis 5 Halogenatomen, Aminocarbonyl oder Aminocarbonyl-C₁-C₄-alkyl steht,
R¹³ für Wasserstoff, Halogen, Cyano, C₁-C₄-Alkyl, C₁-C₄-Alkoxy oder C₁-C₄ Alkylthio steht,
R¹⁴ für Wasserstoff, C₁-C₄-Alkyl, Hydroxy-C₁-C₄-alkyl, C₂-C₆-Alkenyl, C₃-C₆ Cycloalkyl, C₁-C₄-Alkylthio-C₁-C₄-alkyl, C₁-C₄-Alkoxy-C₁-C₄-alkyl, C₁-C₄ Halogenalkyl, C₁-C₄-Halogenalkylthio-C₁-C₄-alkyl, C₁-C₄-Halogenalkoxy C₁-C₄-alkyl mit jeweils 1 bis 5 Halogenatomen, oder Phenyl steht,
oder A für den Rest der Formel (A2) 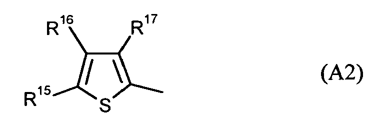 steht, in welcher
R¹⁵ und R¹⁶ unabhängig voneinander für Wasserstoff, Halogen, C₁-C₄-Alkyl oder C₁ C₄-Halogenalkyl mit 1 bis 5 Halogenatomen steht,
R¹⁷ für Halogen, Cyano oder C₁-C₄-Alkyl, oder C₁-C₄-Halogenalkyl oder C₁-C₄ Halogenalkoxy mit jeweils 1 bis 5 Halogenatomen steht,
oder A für den Rest der Formel (A3) 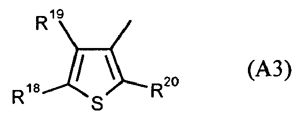 steht, in welcher
R¹⁸ und R¹⁹ unabhängig voneinander für Wasserstoff, Halogen, C₁-C₄-Alkyl oder C₁ C₄-Halogenalkyl mit 1 bis 5 Halogenatomen steht,
R²⁰ für Wasserstoff, Halogen, C₁-C₄-Alkyl oder C₁-C₄-Halogenalkyl mit 1 bis 5 Halogenatomen steht,
oder A für den Rest der Formel (A4) 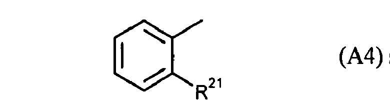 steht, in welcher
R²¹ für Wasserstoff, Halogen, Hydroxy, Cyano, C₁-C₆-Alkyl, C₁-C₄ Halogenalkyl, C₁-C₄-Halogenalkoxy oder C₁-C₄-Halogenalkylthio mit jeweils 1 bis 5 Halogenatomen steht,
oder A für den Rest der Formel (A5) 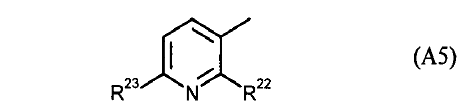 steht, in welcher
R²² für Halogen, Hydroxy, Cyano, C₁-C₄-Allcyl, C₁-C₄-Alkoxy, C₁-C₄-Alkylthio, C₁-C₄-Halogenalkyl, C₁-C₄-Halogenalkylthio oder C₁-C₄-Halogenalkoxy mit jeweils 1 bis 5 Halogenatomen steht,
R²³ für Wasserstoff, Halogen, Cyano, C₁-C₄-Alkyl, C₁-C₄-Alkoxy, C₁-C₄ Alkylthio, C₁-C₄-Halogenalkyl, C₁-C₄-Halogenalkoxy mit jeweils 1 bis 5 Halogenatomen, C₁-C₄-Alkylsulphinyl oder C₁-C₄-Alkylsulphonyl steht,
oder A für den Rest der Formel (A6) 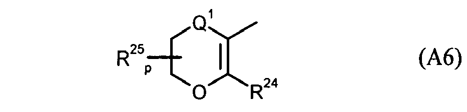 steht, in welcher
R²⁴ für C₁-C₄-Alkyl oder C₁-C₄-Halogenalkyl mit 1 bis 5 Halogenatomen steht,
R²⁵ für C₁-C₄-Alkyl steht,
Q¹ für S (Schwefel), O (Sauerstoff) SO, SO₂ oder CH₂ steht,
p für 0, 1 oder 2, wobei R²⁵ für identische oder verschiedene Reste steht, wenn p für 2 steht,
oder A für den Rest der Formel (A7) 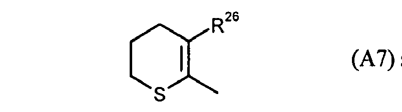 steht, in welcher
R²⁶ für C₁-C₄-Alkyl oder C₁-C₄-Halogenalkyl mit 1 bis 5 Halogenatomen steht,
oder A für den Rest der Formel (A8) 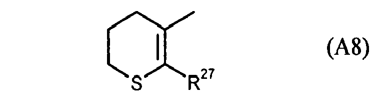 steht, in welcher
R²⁷ für C₁-C₄-Alkyl oder C₁-C₄-Halogenalkyl mit 1 bis 5 Halogenatomen steht,
oder A für den Rest der Formel (A9) 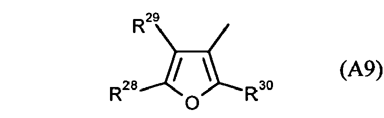 steht, in welcher
R²⁸ und R²⁹ unabhängig voneinander für Wasserstoff, Halogen, Amino, C₁-C₄-Alkyl oder C₁-C₄-Halogenalkyl mit 1 bis 5 Halogenatomen stehen,
R³⁰ für Wasserstoff, Halogen, C₁-C₄-Alkyl oder C₁-C₄-Halogenalkyl mit 1 bis 5 Halogenatomen steht,
oder A für den Rest der Formel (A10) 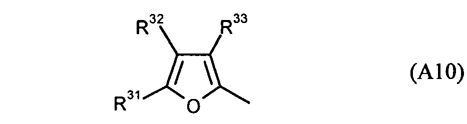 steht, in welcher
R³¹ und R³² unabhängig voneinander für Wasserstoff, Halogen, Amino, Nitro, C₁-C₄-Alkyl oder C₁-C₄-Halogenalkyl having 1 bis 5 Halogenatomen stehen,
R³³ für Wasserstoff, Halogen, C₁-C₄-Alkyl oder C₁-C₄-Halogenalkyl mit 1 bis 5 Halogenatomen steht,
oder A für den Rest der Formel (A11) 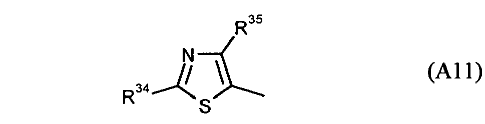 steht, in welcher
R³⁴ für Wasserstoff, Halogen, Amino, C₁-C₄-Alkylamino, Di-(C₁-C₄-alkyl)amino, Cyano, C₁-C₄-Alkyl oder C₁-C₄-Halogenalkyl mit 1 bis 5 Halogenatomen steht,
R³⁵ für Halogen, C₁-C₄-Alkyl oder C₁-C₄-Halogenalkyl mit 1 bis 5 Halogenatomen steht,
oder A für den Rest der Formel (A12) 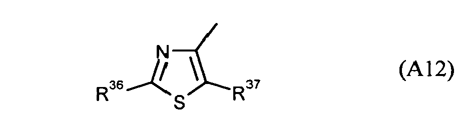 steht, in welcher
R³⁶ für Wasserstoff, Halogen, Amino, C₁-C₄-Alkylamino, Di-(C₁-C₄ alkyl)amino, Cyano, C₁-C₄-Alkyl oder C₁-C₄-Halogenalkyl mit 1 bis 5 Halogenatomen steht,
R³⁷ für Halogen, C₁-C₄-Alkyl oder C₁-C₄-Halogenalkyl mit 1 bis 5 Halogenatomen steht,
oder A für den Rest der Formel (A13) 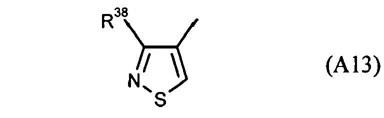 steht, in welcher
R³⁸ für Halogen, C₁-C₄-Alkyl oder C₁-C₄-Halogenalkyl mit 1 bis 5 Halogenatomen steht,
oder
A für den Rest der Formel (A14) 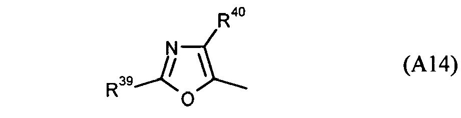 steht, in welcher
R³⁹ für Wasserstoff oder C₁-C₄-Alkyl steht,
R⁴⁰ für Halogen oder C₁-C₄-Alkyl steht,
oder
A für den Rest der Formel (A15) 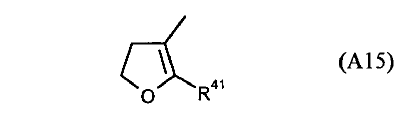 steht, in welcher
R⁴¹ für C₁-C₄-Alkyl oder C₁-C₄-Halogenalkyl mit 1 bis 5 Halogenatomen steht,
oder A für den Rest der Formel (A16) 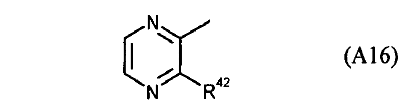 steht, in welcher
R⁴² für Wasserstoff, Halogen, C₁-C₄-Alkyl oder C₁-C₄-Halogenalkyl mit 1 bis 5 Halogenatomen steht,
oder A für den Rest der Formel (A17) 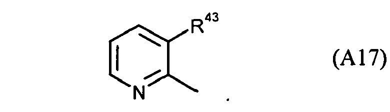 steht, in welcher
R⁴³ für Halogen, Hydroxy, C₁-C₄-Alkyl, C₁-C₄-Alkoxy, C₁-C₄-Alkylthio, C₁-C₄ Halogenalkyl, C₁-C₄-Halogenallcylthio oder C₁-C₄-Halogenalkoxy mit jeweils 1 bis 5 Halogenatomen steht,
oder A für den Rest der Formel (A18) 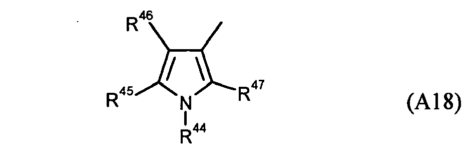 steht, in welcher
R⁴⁴ für Wasserstoff, Cyano, C₁-C₄-Alkyl, C₁-C₄-Halogenalkyl mit 1 bis 5 Halogenatomen, C₁-C₄-Alkoxy-C₁-C₄-alkyl, Hydroxy-C₁-C₄-alkyl, C₁-C₄ Alkylsulfonyl, Di(C₁-C₄-alkyl)aminosulfonyl, C₁-C₆-Alkylcarbonyl oder jeweils gegebenenfalls substituiertes Phenylsulfonyl oder Benzoyl steht,
R⁴⁵ für Wasserstoff, Halogen, C₁-C₄-Alkyl oder C₁-C₄-Halogenalkyl mit 1 bis 5 Halogenatomen steht,
R⁴⁶ für Wasserstoff, Halogen, Cyano, C₁-C₄-Alkyl oder C₁-C₄-Halogenalkyl mit 1 bis 5 Halogenatomen steht,
R⁴⁷ für Wasserstoff, Halogen, C₁-C₄-Alkyl oder C₁-C₄-Halogenalkyl mit 1 bis 5 Halogenatomen steht,
oder A für den Rest der Formel (A19) 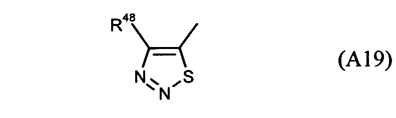 steht, in welcher
R⁴⁸ für C₁-C₄-Alkyl steht.
2. Verfahren zum Herstellen der 2-Alkyl-cycloalk(en)yl-carboxamide der Formel (I) gemäß Anspruch 1, **dadurch gekennzeichnet, dass** man (a) Carbonsäure-Derivate der Formel (II) 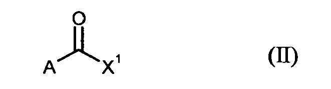 in welcher A die in Anspruch 1 angegebenen Bedeutungen hat und
X¹ für Halogen oder Hydroxy steht,
mit Anilin-Derivaten der Formel (III) 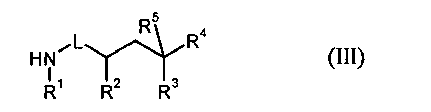 in welcher R¹, L, R², R³, R⁴ und R⁵ die in Anspruch 1 angegebenen Bedeutungen haben, gegebenenfalls in Gegenwart eines Katalysators, gegebenenfalls in Gegenwart eines Kondensationsmittels, gegebenenfalls in Gegenwart eines Säurebindemittels und gegebenenfalls in Gegenwart eines Verdünnungsmittels umsetzt, oder (b) 2-Alkyl-cycloalk(en)yl-carboxamide der Formel (I-a) 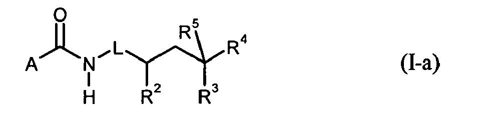 in welcher L, R², R³, R⁴, R⁵ und A die in Anspruch 1 angegebenen Bedeutungen haben, mit Halogeniden der Formel (IV)
R^{1A}-X² (IV)
in welcher
R^{1A} für C₁-C₈-Alkyl, C₁-C₆-Alkylsulfinyl, C₁-C₆-Alkylsulfonyl, C₁-C₄-Alkoxy-C₁ C₄-allcyl, C₃-C₈-Cycloalkyl; C₁-C₆-Halogenalkyl, C₁-C₄-Halogenalkylthio, C₁-C₄-Halogenalkylsulfinyl, C₁-C₄-Halogenalkylsulfonyl, Halogen-C₁-C₄ alkoxy-C₁-C₄-alkyl, C₃-C₈-Halogencycloalkyl mit jeweils 1 bis 9 Fluor-, Chlor- und/oder Bromatomen; Formyl, Formyl-C₁-C₃-alkyl, (C₁-C₃-Alkyl)-carbonyl-C₁-C₃-alkyl, (C₁-C₃-Alkoxy)carbonyl-C₁-C₃-alkyl; Halogen-(C₁-C₃ alkyl)carbonyl-C₁-C₃-alkyl, Halogen-(C₁-C₃-alkoxy)carbonyl-C₁-C₃-alkyl mit jeweils 1 bis 13 Fluor-, Chlor- und/oder Bromatomen; (C₁-C₈-Alkyl)carbonyl, (C₁-C₈-Alkoxy)carbonyl, (C₁-C₄-Alkoxy-C₁-C₄-alkyl)-carbonyl, (C₃-C₈-Cycloalkyl)carbonyl; (C₁-C₆-Halogenalkyl)carbonyl, (C₁-C₆ Halogenalkoxy)carbonyl, (Halogen-C₁-C₄-alkoxy-C₁-C₄-alkyl)carbonyl, (C₃C₈-Halogencycloalkyl)carbonyl mit jeweils 1 bis 9 Fluor-, Chlor- und/oder Bromatomen; oder -C(=O)C(=O)R⁶, -CONR⁷R⁸ oder -CH₂NR⁹R¹⁰ steht,
R⁶, R⁷, R⁸, R⁹ und R¹⁰ die in Anspruch 1 angegebenen Bedeutungen haben,
X² für Chlor, Brom oder Iod steht, in Gegenwart einer Base und in Gegenwart eines Verdünnungsmittels umsetzt.

**3.** Mittel zur Bekämpfung unerwünschter Mikroorganismen, **gekennzeichnet durch** einen Gehalt an mindestens einem 2-Alkyl-cycloalk(en)yl-carboxamid der Formel (I) gemäß Anspruch 1 neben Streckmitteln und/oder oberflächenaktiven Stoffen.

**4.** Verwendung von 2-Alkyl-cycloalk(en)yl-carboxamiden der Formel (I) gemäß Anspruch 1 zur Bekämpfung unerwünschter Mikroorganismen.

**5.** Verfahren zur Bekämpfung unerwünschter Mikroorganismen, **dadurch gekennzeichnet, dass** man 2-Alkyl-cycloalk(en)yl-carboxamide der Formel (I) gemäß Anspruch 1 auf die Mikroorganismen und/oder deren Lebensraum ausbringt.

**6.** Verfahren zur Herstellung von Mitteln zur Bekämpfung unerwünschter Mikroorganismen, **dadurch gekennzeichnet, dass** man 2-Alkyl-cycloalk(en)yl-carboxamide der Formel (I) gemäß Anspruch 1 mit Streckmitteln und/oder oberflächenaktiven Stoffen vermischt.
